# EUROPEAN PATENT APPLICATION

(11) **EP 4 556 887 A1**
(43) Date of publication of application: **21.05.2025**
(21) Application number: 23838984.5
(22) Date of filing: 12.07.2023
(51) Int. Cl.: G01N 21/01, G01N 21/78, G01N 33/50, E03D 9/00, E03D 11/00, A47K 13/00, A61B 10/00

(54) **HEALTH MONITORING APPARATUS AND HEALTH MONITORING TOILET**

(30) Priority: 13.07.2022 CN 202221802415 U; 17.08.2022 CN 202222170357 U; 02.09.2022 CN 202222344083 U; 29.11.2022 CN 202223180273 U; 23.12.2022 CN 202223457582 U; 23.04.2023 CN 202320933803 U
(71) Applicant: XIAMEN R&T PLUMBING TECHNOLOGY CO., LTD., Xiamen, Fujian 361028 (CN)
(72) Inventor: ZHONG, Zhijun, Xiamen, Fujian 361028 (CN); ZHANG, Zulian, Xiamen, Fujian 361028 (CN); LIN, Jian, Xiamen, Fujian 361028 (CN); HUANG, Candong, Xiamen, Fujian 361028 (CN); LIU, Zhipeng, Xiamen, Fujian 361028 (CN)
(74) Representative: Loo, Chi Ching
(86) International application number: PCT/CN2023/107020
(87) International publication number: WO 2024/012494

(57) **Abstract**

Disclosed are a health monitoring apparatus and a health monitoring toilet, belonging to the technical field of urine test devices. The health monitoring apparatus comprises a monitoring module, a plugging element, and a transmission member capable of being in transmission fit with the monitoring module or the plugging element. The monitoring module comprises a monitoring element capable of monitoring a liquid to be monitored, and the plugging element has a sealing portion for plugging a monitoring hole of the toilet. During monitoring, the transmission member drives the monitoring module, such that the monitoring element extends out of the monitoring hole for monitoring. When no monitoring is performed, the transmission member drives the plugging element, such that the sealing portion is in sealing fit with the monitoring hole. The same transmission unit, which is in transmission fit with the monitoring module or the plugging element respectively, drives the monitoring module to extend out during monitoring, and drives the plugging element to extend out to seal the monitoring hole when no monitoring is performed. Therefore, the normal use function of the toilet is not affected, the monitoring module is prevented from being contaminated, and the health monitoring apparatus has a simple structure and reliable functions.

## Description

### TECHNICAL FIELD

The present invention relates to the technical field of urine test devices, and more specifically, to a health monitoring apparatus and a health monitoring toilet.

### BACKGROUND

In modern society, people are paying more and more attention to health. Urine is one of the important metabolites that reflect the health status of the body, and urine test is developing from relying on specific places (hospitals) and specific personnel (medical staff) to ordinary toilet locations and being measurable by everyone. Therefore, toilets with urine test functions have emerged. However, the existing urine test toilets still have many problems that need to be improved.

### SUMMARY

One of the objectives of the present invention is to provide a health monitoring apparatus, where the same transmission unit, which is in transmission fit with a monitoring module or a plugging element respectively, drives the monitoring module to extend out during monitoring, and drives the plugging element to extend out to seal a monitoring hole when no monitoring is performed, so the normal use function of the toilet is not affected, and the monitoring module is prevented from being contaminated.

In order to achieve the above objective, the present invention provides the following first technical solution:

A health monitoring apparatus for fitting with a monitoring hole of a toilet includes:
a monitoring module comprising a monitoring element capable of monitoring a liquid to be monitored;
a plugging element with a sealing portion for plugging the monitoring hole; and
a transmission unit with a transmission member capable of being in transmission fit with the monitoring module or the plugging element, where during monitoring, the transmission member drives the monitoring module, such that the monitoring element extends out of the monitoring hole for monitoring; when no monitoring is performed, the transmission member drives the plugging element, such that the sealing portion is in sealing fit with the monitoring hole.

Compared with the prior art, the beneficial effects of the first technical solution include at least the following:
1. The health monitoring apparatus provided by the present invention includes the monitoring module, the plugging element, and the transmission member capable of being in transmission fit with the monitoring module or the plugging element; the monitoring module includes the monitoring element capable of monitoring the liquid to be monitored, and the plugging element has the sealing portion for plugging the monitoring hole of the toilet; during monitoring, the transmission member drives the monitoring module, such that the monitoring element extends out of the monitoring hole for monitoring; when no monitoring is performed, the transmission member drives the plugging element, such that the sealing portion is in sealing fit with the monitoring hole. The same transmission unit, which is in transmission fit with the monitoring module or the plugging element respectively, drives the monitoring module to extend out during monitoring, and drives the plugging element to extend out to seal the monitoring hole when no monitoring is performed. Therefore, the normal use function of the toilet is not affected, the monitoring module is prevented from being contaminated, and the health monitoring apparatus has a simple structure and reliable functions.
2. The health monitoring apparatus provided by the present invention further includes a container body and a driving unit, where the container body has a plurality of accommodating channels inside, and the driving unit includes a driving member in transmission fit with the container body; the driving member drives the container body to move, such that at least one of the accommodating channels corresponds to the monitoring hole; the monitoring module is arranged in the accommodating channel in an extensible and retractable manner; therefore, the overall structure is compact, and the cost is low. The container body moves to change the position of the accommodating channel, such that the accommodating channel corresponding to the monitoring hole changes with the monitoring module arranged therein in the extensible and retractable manner; therefore, the health monitoring apparatus is skillful, simple, and easy to operate. The fit between the driving mechanism and the transmission mechanism can achieve automatic replacement of the monitoring module, so the health monitoring apparatus is convenient to use and does not require manual replacement.

The second objective of the present invention is to provide a health monitoring apparatus, which has the advantages of convenient replacement of a monitoring module, as well as no water leakage and higher safety because a plugging element is still plugged in a channel when the monitoring module is replaced.

To achieve the above objective, the present invention provides the following second technical solution:

A health monitoring apparatus includes a fixing frame, a plugging element, and a monitoring module; the fixing frame includes a mounting shell, a container body is detachably connected inside the mounting shell, the container body is provided with a first accommodating chamber for accommodating the plugging element and at least one second accommodating chamber for accommodating the monitoring module, the fixing frame is further provided with a channel corresponding to a monitoring hole of a toilet, the end of the channel near the monitoring hole is open, a transmission unit for driving the plugging element and the monitoring module to move is further provided on the fixing frame, a driving unit for driving the container body to move is further provided on the fixing frame, and the container body is further provided with a yielding portion in yielding fit with the transmission unit; when the container body moves to the first accommodating chamber to correspond to the channel, the transmission unit can drive the plugging element to be limited in the channel, and the container body is in yielding fit with the transmission unit through the yielding portion, such that the container body can be separated from the mounting shell.

Compared with the prior art, the beneficial effects of the second technical solution include at least the following:
1. By configuring the container body, the first accommodating chamber, the transmission member, and the driving unit, when the monitoring module needs to be replaced, the container body is directly removed, so the operation is easy; in addition, when the monitoring module is replaced, the plugging element is still plugged in the channel, so no water leaks, and the safety is higher;
2. By configuring a rear cover assembly, a first clamping portion, and a second clamping portion, when the driving unit drives the container body to rotate, the entire container assembly is fixed relative to the mounting shell, thereby improving the stability of the container assembly and avoiding detachment.

The third objective of the present invention is to provide a health monitoring apparatus, where the fit between a limit member for state switching and a stop portion avoids retraction of a movable member at a designated position, thereby at least to some extent overcoming one or more problems caused by limitations and defects in related technologies.

To achieve the above objective, the present invention provides the following third technical solution:

A health monitoring apparatus, arranged on a toilet, includes:
a fixing frame arranged on the toilet, the fixing frame including a channel for extracting a sample to be monitored;
a movable member, the movable member being movable relative to the channel; and
a limit member arranged on the fixing frame, the limit member including a limiting state and at least one yielding state, and the limit member being capable of switching between the limiting state and the at least one yielding state;
where the movable member includes at least one stop portion, the limit member switches to the limiting state, the limit member fits with the at least one stop portion, and the movable member is limited at a first position; when the limit member switches to the yielding state, the limit member disengages from the fit with the stop portion, and the movable member is released from the limit.

Compared with the prior art, the beneficial effects of the third technical solution include at least the following:
1. In the case where the plugging element is movable, the fit between the limit member capable of switching states and the at least one stop portion provided on the plugging element can limit the movable member from retracting in the first position, thereby avoiding the problems of poor sealing and water leakage of the monitoring apparatus due to the retraction of the movable member at the designated position while satisfying the monitoring function;
2. During the retraction of the monitoring element to the monitoring kit, the limit member switches to the limiting state to limit the monitoring kit. After the monitoring element retracts to the preset position, the limit member switches to the yielding state to yield the monitoring kit. The precise positioning of the monitoring element is achieved by the limit member, thereby preventing the monitoring result from being affected by the monitoring element that does not retract in place.

The fourth objective of the present invention is to provide a health monitoring apparatus, where a main control unit monitors positioning duration of a sealing portion in a monitoring hole, which can periodically drive a plugging element to move, thereby solving the problem of increased starting friction caused by long-term disuse of the plugging element, reducing starting friction, and making starting effortless, with a simple structure, low cost and long lifespan.

To achieve the above objective, the present invention provides the following fourth technical solution:

A health monitoring apparatus for fitting with a monitoring hole of a toilet includes:
a monitoring module comprising a monitoring element capable of extending out of the monitoring hole to monitor a liquid to be monitored;
a plugging element with a sealing portion for plugging the monitoring hole; and
a transmission unit including a driving circuit and a transmission member, where the driving circuit is used for controlling the transmission member to drive the plugging element; and
a main control unit capable of monitoring positioning duration of the sealing portion in the monitoring hole, where the main control unit is electrically connected to the driving circuit; when the main control unit monitors that the positioning duration exceeds preset duration, the driving circuit controls the transmission member to drive the plugging element to move a preset distance.

Compared with the prior art, the beneficial effects of the fourth technical solution include at least the following:
1. The main control unit monitors the positioning duration of the sealing portion positioned in the monitoring hole, which can periodically drive the plugging element to move, thereby solving the problem of increased starting friction caused by long-term disuse of the plugging element, reducing starting friction, and making starting effortless, with a simple structure, low cost and long lifespan.

The fifth objective of the present invention is to provide a health monitoring toilet, which can achieve non-contact automatic monitoring, solve the contamination of existing excrement on a device, and better maintain the hygiene of the device.

To achieve the above objective, the present invention provides the following fifth technical solution:

A health monitoring toilet includes:
a toilet having a toilet bowl, a bottom of which is provided with a monitoring hole;
a monitoring element penetrating into the toilet bowl from the monitoring hole to monitor excrement;
a camera module for capturing towards the toilet bowl; and
an analysis module connected to the camera module, where information collected by the camera module is transmitted to the analysis module for processing and analysis;
where the monitoring element extends out from the monitoring hole to monitor excrement in the toilet bowel, and the camera module captures a monitoring result of the monitoring element and transmits the monitoring result to the analysis module for processing and analysis.

Compared with the prior art, the beneficial effects of the fifth technical solution include at least the following:
1. The camera module is used for capturing towards the toilet bowl and transmitting the monitoring result to the analysis module for data processing and analysis to obtain the human health status, thereby achieving non-contact automatic monitoring, solving the contamination of existing excrement on a device, and better maintaining the hygiene of the device;
2. The position of a lens of the camera module can be changed by a voice coil motor in the camera module, to achieve automatic focusing, making the captured images clearer and reducing the errors of analysis on blurry images by the analysis module;
3. By configuring a lighting source, the results of monitoring by the monitoring element are clearer during capturing, and the clarity of images is improved.

The sixth objective of the present invention is to provide a health monitoring toilet, which enables a user to monitor excrement at home simply, reduces the dilution of excrement by water in a water seal area, can ensure the stability and accuracy of monitoring results, and effectively improves the monitoring efficiency and effectiveness.

To achieve the above objective, the present invention provides the following sixth technical solution:

A health monitoring toilet includes:
a toilet including a toilet bowl and a drain pipe, where the toilet bowl has a water seal, and water from the water seal can be discharged to the drain pipe;
a health monitoring apparatus for monitoring a sample to be monitored;
a water level sensor for monitoring a water level of the water seal; and
a control unit electrically connected to the water level sensor and the health monitoring apparatus, where the control unit can control the health monitoring apparatus to turn on based on a monitoring signal of the water level sensor.

Compared with the prior art, the beneficial effects of the sixth technical solution include at least the following:
1. The control unit can control the health monitoring apparatus to turn on based on the monitoring signal of the water level sensor, thereby reducing the dilution of excrement by water in the water seal area, ensuring the stability and accuracy of monitoring results, and effectively improving the monitoring efficiency and effectiveness;
2. The control unit is further electrically connected to a control valve. After the control unit receives a first water level signal and the health monitoring apparatus is turned off, the control unit controls the control valve to open. This solution involves only one sensor, which can determine whether to turn on the health monitoring apparatus for monitoring and whether to open an electromagnetic valve for water supply, thereby saving costs.

### BRIEF DESCRIPTION OF THE DRAWINGS

FIG. 1 is a three-dimensional schematic diagram of a monitoring apparatus provided in Embodiment 1;
FIG. 2 is a cross-sectional view of the monitoring apparatus provided in Embodiment 1 in one direction;
FIG. 3 is a three-dimensional schematic diagram of a container body of the monitoring apparatus provided in Embodiment 1;
FIG. 4 is a three-dimensional schematic diagram showing that the container body of the monitoring apparatus provided in Embodiment 1 is connected to a driving member;
FIG. 5 is a schematic diagram of another monitoring apparatus provided in Embodiment 1;
FIG. 6 is a cross-sectional view of a toilet provided in Embodiment 1 when a plugging element extends out;
FIG. 7 is a schematic diagram of a health monitoring apparatus in FIG. 6;
FIG. 8 is a cross-sectional view of the toilet provided in Embodiment 1 when a monitoring kit does not extend out at the beginning of monitoring;
FIG. 9 is a schematic diagram of the health monitoring apparatus in FIG. 8;
FIG. 10 is a cross-sectional view of the toilet provided in Embodiment 1 when the monitoring kit extends out during monitoring;
FIG. 11 is a schematic diagram of the health monitoring apparatus in FIG. 10;
FIG. 12 is an overall schematic diagram of a health monitoring apparatus provided in Embodiment 2;
FIG. 13 is a cross-sectional view when a plugging element plugs a channel in Embodiment 2;
FIG. 14 is a cross-sectional view when a monitoring element extends out of the channel in Embodiment 2;
FIG. 15 is a schematic diagram when a limit portion is in clamping fit with a fit portion in Embodiment 2;
FIG. 16 is a schematic diagram when the limit portion separates from the fit portion in Embodiment 2;
FIG. 17 is an enlarged schematic diagram of part A in FIG. 12;
FIG. 18 is an overall schematic diagram of some container components provided in Embodiment 2;
FIG. 19 is an overall schematic diagram of a health monitoring apparatus provided in Embodiment 3;
FIG. 20 is a schematic structural diagram of a shell and a limit member provided in Embodiment 3;
FIG. 21 is a schematic structural diagram of a movable member provided in Embodiment 3;
FIG. 22 is a schematic structural diagram of a plugging element provided in Embodiment 3;
FIG. 23 is a schematic structural diagram of a monitoring module provided in Embodiment 3;
FIG. 24 is a schematic structural diagram of the limit member and the movable member provided in Embodiment 3 in a limiting state;
FIG. 25 is a schematic structural diagram of the limit member and the movable member provided in Embodiment 3 in a yielding state;
FIG. 26 is a schematic structural diagram of the limit member and the movable member provided in Embodiment 3 in another yielding state;
FIG. 27 is a first cross-sectional view of a health monitoring apparatus provided in Embodiment 4;
FIG. 28 is a second cross-sectional view of the health monitoring apparatus provided in Embodiment 4;
FIG. 29 is a schematic structural diagram of a health monitoring toilet provided in Embodiment 5;
FIG. 30 is a schematic diagram of a work process provided in Embodiment 5;
FIG. 31 is a cross-sectional view of a health monitoring toilet provided in Embodiment 6;
FIG. 32 is an enlarged schematic diagram of part B in FIG. 31;
FIG. 33 is a cross-sectional view of a health monitoring toilet provided in Embodiment 7;
FIG. 34 is an enlarged schematic diagram of part C in FIG. 33;
FIG. 35 is a cross-sectional view of a health monitoring toilet provided in Embodiment 8; and
FIG. 36 is an enlarged schematic diagram of part D in FIG. 35.

### LIST OF REFERENCES:

1. Monitoring module; 101. Bracket; 1011. Mounting groove; 1012. Sealing member; 102. Test paper; 103. Monitoring kit; 1031. Inner cavity; 1032. Through channel;
2. Plugging element; 201. Sealing portion;
3. Transmission unit; 301. Rack; 302. Gear set; 303. Driving motor;
4. Container body; 401. Accommodating channel; 402. First accommodating chamber; 403. Second accommodating chamber;
5. Driving unit; 501. Driving member; 502. Limit groove; 503. First power member;
6. Insertion hole; 601. Limit rib;
7. Fixing frame; 701. Mounting shell; 702, Channel;
8. Toilet; 801. Monitoring hole; 802. Accommodating chamber; 803. Toilet bowl;
9. Limit portion; 10. Fit portion; 11. First clamping portion;
12. Second clamping portion; 1201. Operation block; 1202. Clamping block;
13. Yielding portion; 14. Slide; 15. Second slot; 20. Guide groove; 21. Limit member; 22. Plugging plate; 23. Yielding groove; 24. Movable member; 25. Stop portion; 26. Drain pipe; 27. Water seal; 28. Nozzle; 29. Camera module; 30. Base; 32. First mounting hole; 33. Second mounting hole; 34. Water level sensor; 35. Spray unit; 36. Spray channel; 37. Control valve; 38. Rear cover assembly; 39. Shell; 3901. Second rack; 40. Second gear set; 41. Second motor; 42. Health monitoring apparatus.

### DETAILED DESCRIPTION

Hereinafter, the present invention will be further described in detail with reference to the accompanying drawings.

### Embodiment 1:

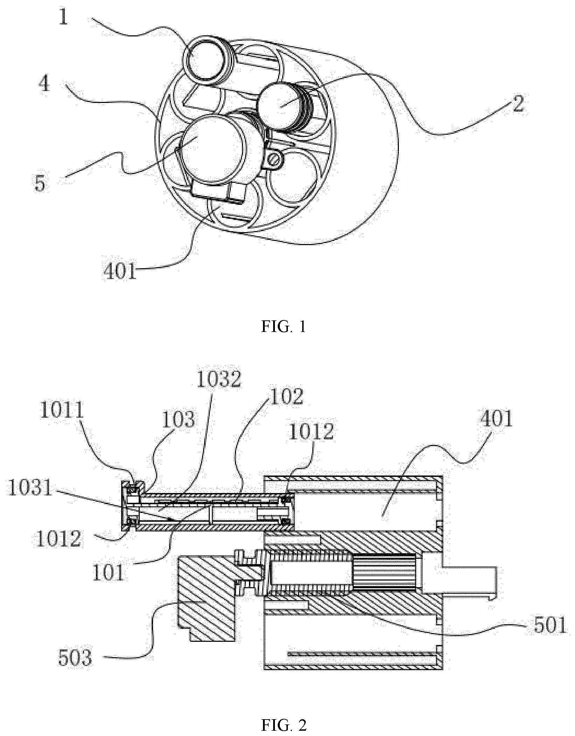

A health monitoring apparatus, as shown in FIG. 1 to FIG. 4, is used for fitting with a monitoring hole 801 of a toilet 8. The monitoring apparatus includes a monitoring module 1, a plugging element 2, and a transmission unit 3. The monitoring module 1 includes a monitoring element capable of monitoring a liquid to be monitored (urine), the plugging element 2 has a sealing portion 201 for plugging the monitoring hole 801, and the transmission unit 3 has a transmission member capable of being in transmission fit with the monitoring module 1 or the plugging element 2. During monitoring, the transmission member drives the monitoring module 1, such that the monitoring element extends out of the monitoring hole 801 for monitoring. When no monitoring is performed, the transmission member drives the plugging element 2, such that the sealing portion 201 is in sealing fit with the monitoring hole 801. The same transmission unit 3, which is in transmission fit with the monitoring module 1 or the plugging element 2 respectively, drives the monitoring module 1 to extend out during monitoring, and drives the plugging element 2 to extend out to seal the monitoring hole 801 when no monitoring is performed. Therefore, the normal use function of the toilet 8 is not affected, the monitoring module 1 is prevented from being contaminated, and the health monitoring apparatus has a simple structure and reliable functions.

In this embodiment, the health monitoring apparatus may further include a container body 4 and a driving unit 5, where the container body 4 has a plurality of accommodating channels 401 inside, the monitoring module 1 is mounted in at least one of the accommodating channels 401, and the driving unit 5 includes a driving member 501 in transmission fit with the container body 4; the driving member 501 drives the container body 4 to move, such that at least one of the accommodating channels 401 corresponds to the monitoring hole 801; the monitoring module 1 is arranged in the accommodating channel 401 in an extensible and retractable manner.

Specifically, the monitoring module 1 is in sliding fit with the accommodating channel 401, such that the monitoring module 1 can extend out of the accommodating channel 401 or retract into the accommodating channel 401. The monitoring module 1 may have a structure with an inner cavity 1031, and the inner cavity 1031 is used for storing the monitoring element. The monitoring element includes a monitoring tool, such as test paper 102. Alternatively, the monitoring tool may be other common (urine) monitoring tools, such as a monitoring card or a monitoring pen. The test paper 102 may be exposed after the monitoring module 1 extends out of the accommodating channel 401, to monitor the liquid to be monitored (urine).

Specifically, the container body 4 may have a shell structure, and the accommodating channel 401 includes two opposite ends and side walls connecting the two ends. In some embodiments, the side walls are connected into a whole (refer to annular side walls in FIGs. 1-4), that is, the cross-section of the accommodating channel 401 has a hollow closed shape, such as circular in FIGs. 1-4, or triangular, quadrilateral, pentagonal, hexagonal, octagonal, etc. In other embodiments, the side walls may not be connected to each other, that is, the cross-section of the accommodating channel 401 cannot form a closed shape, such as semi-circular, straight, crescent, etc., as long as the accommodating channels 401 can be independent of each other and be in sliding fit with the monitoring module 1. The monitoring module 1 is mounted in at least one of the accommodating channels 401, that is, at least one accommodating channel 401 is loaded with the monitoring module 1, and the monitoring module 1 is in sliding fit with the accommodating channel 401 to extend out of the accommodating channel 401 or retract into the accommodating channel 401. When at least two accommodating channels 401 are loaded with monitoring modules 1, the user does not need to clean and discard the waste monitoring tool after each monitoring, but discards or replaces the container body 4 after all the monitoring tools are used up.

In one embodiment, as shown in FIG. 2, the monitoring module 1 includes a monitoring kit 103, the monitoring kit 103 has an inner cavity 1031, the monitoring element is arranged in the inner cavity 1031, and two ends of a kit body of the monitoring kit 103 are connected to form a through channel 1032. The monitoring element includes a bracket 101 and test paper 102, the test paper 102 is laid on the bracket 101, and the bracket 101 is connected to the through channel 1032 in a sliding and sealing manner. The test paper 102 is laid on the bracket 101, such that the test paper 102 is in a closed environment before and after use, will not be affected with damp before use, and will not emit odors to affect the environment after use. Specifically, two ends of the bracket 101 are adapted to the height of the through channel 1032. The two ends of bracket 101 are further provided with mounting grooves 1011, a sealing member 1012 is mounted in the mounting groove 1011, and the sealing member 1012 may be a sealing ring, preferably a Y-shaped ring with good sealing property. Because the bracket 101 can slide relative to the through channel 1032, the test paper 102 laid on the bracket 101 can not only follow the monitoring kit 103 to extend out of the accommodating channel 401, but also follow the bracket 101 to extend out of the through channel 1032 of the monitoring kit 103 again, thereby expanding the extensible range of the test paper 102. On the one hand, urine can drip onto the test paper 102 more sufficiently to improve the accuracy of monitoring results, and on the other hand, the sizes of the monitoring kit 103, the accommodating channel 401 and the container body 4 can be reduced, making the health monitoring apparatus more simple and lightweight.

In this embodiment, the driving unit 5 is in transmission fit with the container body 4 to drive the container body 4 to move, such that at least one of the accommodating channels 401 corresponds to the monitoring hole 801. The driving unit 5 may include a driving member 501 and a first power member 503, where the first power member 503 provides driving force for the driving member 501. In one embodiment, the plugging element 2 is arranged in the accommodating channel 401 in an extensible and retractable manner. The monitoring module 1 extends out of the accommodating channel 401 to allow the test paper 102 in its inner cavity 1031 for monitoring. When no monitoring is performed, the plugging element 2 extends out of the accommodating channel 401, and the sealing portion 201 of the plugging element 2 is in sealing fit with the monitoring hole 801 for plugging. Alternatively, in another embodiment, the plugging element 2 may be independently arranged outside the container body 4 (not shown). When no monitoring is performed, the transmission member of the transmission unit 3 drives the plugging element 2, such that he sealing portion 201 is in sealing fit with the monitoring hole 801.

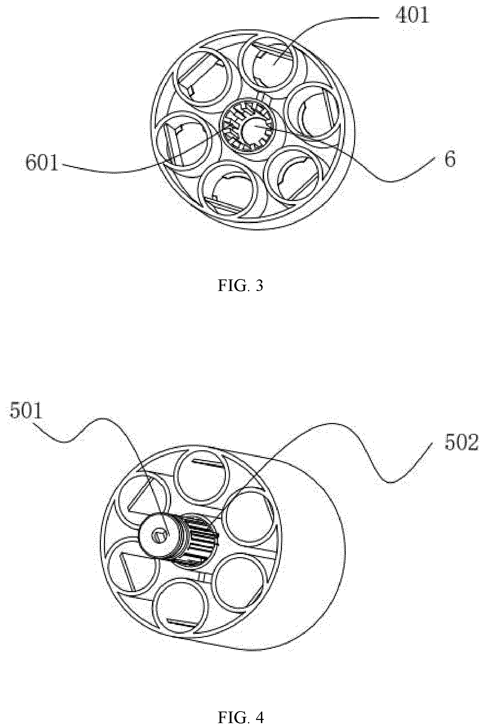

As shown in FIG. 1 to FIG. 4, in a preferred embodiment, the container body 4 is cylindrical, the accommodating channel 401 penetrates along an axis of the container body 4, and the plurality of accommodating channels 401 are arranged sequentially along a circumference of the container body 4. The driving member 501 is a driving shaft, and the first power member 503 is a rotating motor. The container body 4 is provided with an insertion hole 6, and the driving shaft is in limiting fit with the insertion hole 6 circumferentially. More specifically, as shown in FIG. 3 and FIG. 4, limit ribs 601 are provided in the insertion hole 6, the driving shaft is provided with limit grooves 502, and the limit ribs 601 are adapted to the limit grooves 502 to ensure a stable connection between the driving shaft and the container body 4, with good transmission effect and fastening effect. It is easy to conceive that the positions of the limit ribs 601 and the limit grooves 502 can also be interchanged (not shown), that is, the limit grooves 502 are provided in the insertion hole 6, and the limit ribs 601 are provided on the driving shaft. The insertion hole 6 is located in a central position of the container body 4, such that the driving unit 5 is mounted more stably, and it is more stable for the driving unit to move and drive the container body 4 to move. In this embodiment, the container body 4 is rotatably arranged, that is, the driving unit 5 drives the container body 4 to rotate, such that at least one of the accommodating channels 401 corresponds to the monitoring hole 801. Because the container body 4 is cylindrical, its rotation effect is optimal. It is easy to conceive that the container body 4 may alternatively be in the shape of a prism, and the more sides of the prism, the closer the prism is to the cylindrical shape, and the better the effect.

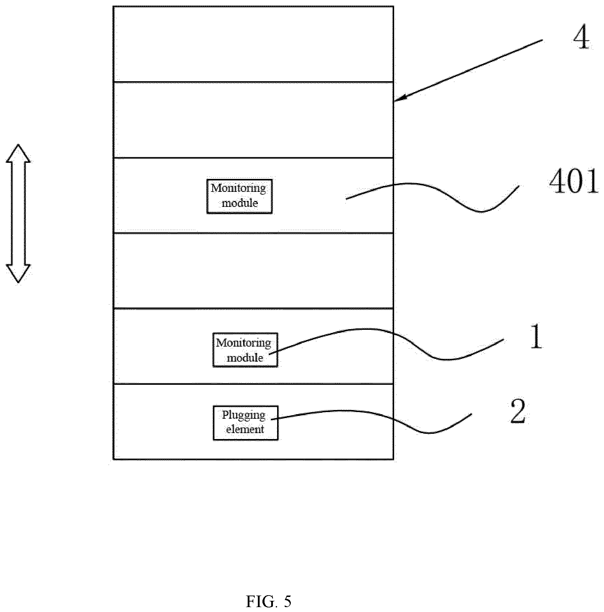

In another embodiment, the container body 4 is arranged in a sliding manner, and the driving unit 5 drives the container body 4 to slide, such that at least one of the accommodating channels 401 corresponds to the monitoring hole 801. Specifically, as shown in FIG. 5, the container body 4 has a plurality of independent accommodating channels 401 inside, and the plurality of accommodating channels 401 are arranged sequentially according to a sliding direction. Specifically, the plurality of accommodating channels 401 are arranged sequentially in a vertical direction, and the driving unit 5 drives the container body 4 to slide up and down in the vertical direction. It is easy to conceive that the plurality of accommodating channels 401 may alternatively be arranged sequentially in a horizontal direction or in other directions, as long as their arrangement direction is consistent with the direction in which the driving unit 5 drives the container body 4 to slide.

In this embodiment, the transmission unit 3 has a transmission member, and the transmission member can extend into or withdraw from the accommodating channel 401 to drive the connected monitoring module 1 or plugging element 2, so as to drive the monitoring module 1 or the plugging element 2 (when the plugging element 2 is arranged in the accommodating channel 401 in an extensible and retractable manner) to extend out of the accommodating channel 401 or retract into the accommodating channel 401.

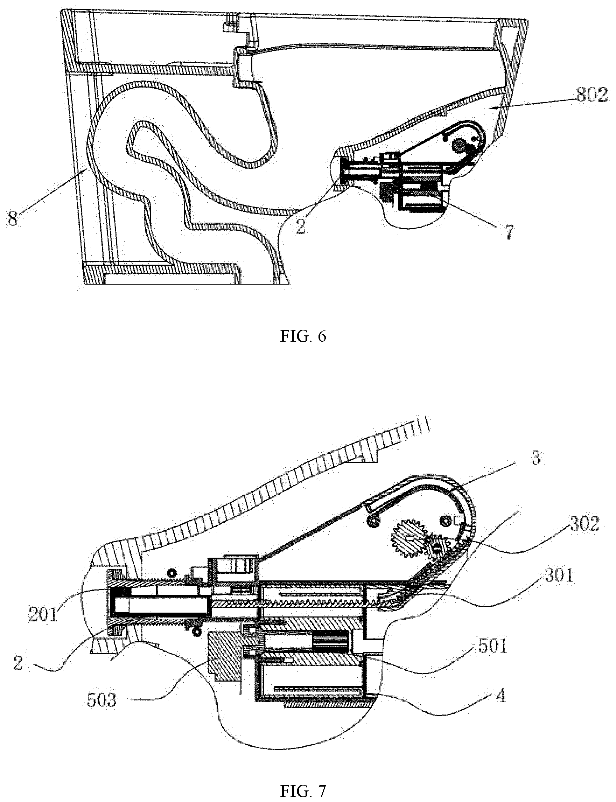

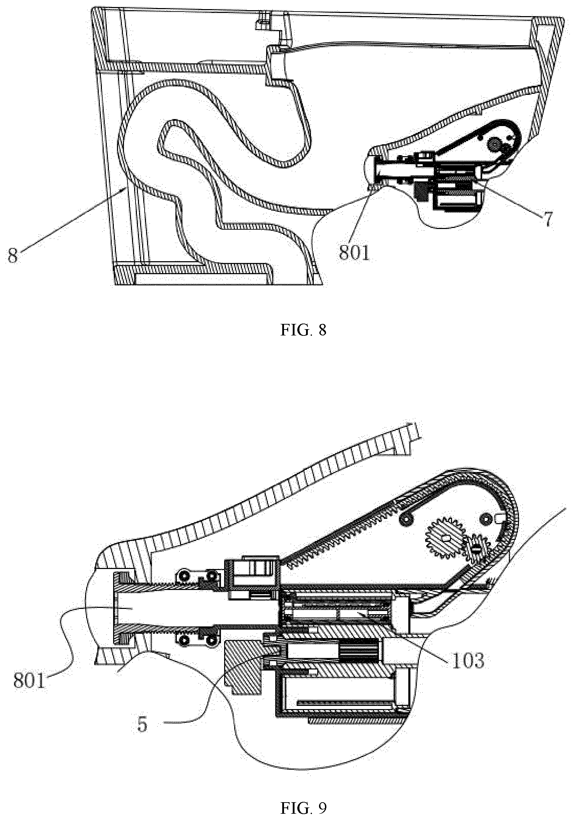

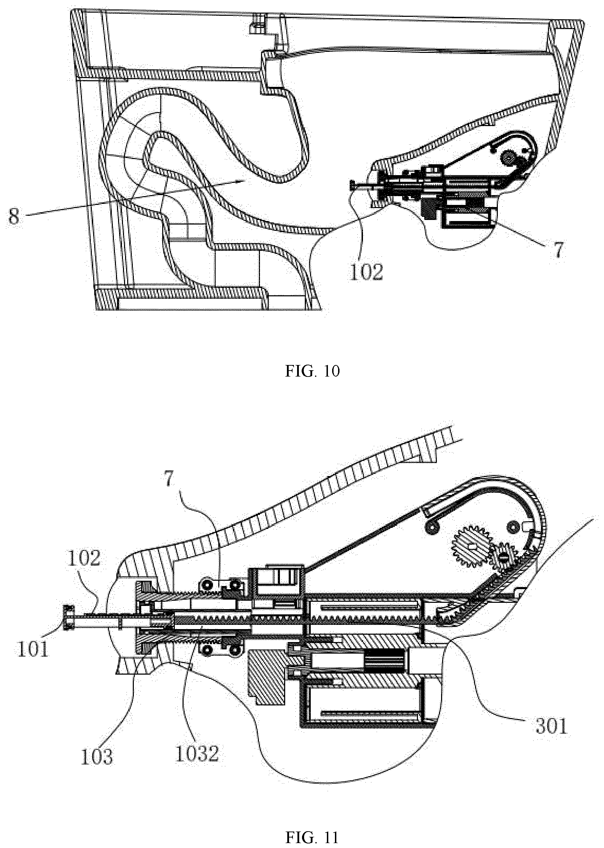

In one embodiment, with reference to FIGs. 6-11, the transmission member is a rack 301, the rack 301 is in separable fit with one end of the monitoring module 1 and/or the plugging element 2, and the transmission unit 3 further includes a gear set 302 meshing with the rack 301 and a driving motor 303 driving the gear set 302.

The aforementioned health monitoring apparatus further includes a fixing frame 7, and the container body 4 is detachably mounted on the fixing frame 7, so as to achieve stable movement (such as rotation or sliding) and easy disassembly. For example, after all the monitoring modules 1 are used up (the used monitoring modules 1 may be discarded immediately or pulled back to the original accommodating channels 401 by the transmission unit 3 after use), the container body 4 can be disassembled and replaced with a new one.

In this embodiment, when the plugging element 2 is arranged in the accommodating channel 401 in an extensible and retractable manner, under the joint action of the driving unit 5 and the transmission unit 3, the container body 4 can move, such that at least one of the accommodating channels 401 corresponds to the monitoring hole 801, thereby achieving the switching of the target accommodating channel 401, and the fit with the monitoring module 1 or the plugging element 2 extending out of the accommodating channel 401 or retracting into the accommodating channel 401. The fit between the driving unit 5 and the transmission unit 3 can achieve automatic replacement/switching of the monitoring module 1 and/or the plugging element 2, so the health monitoring apparatus is convenient to use and does not require manual replacement/switching.

The present invention further provides a toilet 8. As shown in FIGs. 6-11, a monitoring hole 801 is formed on a side wall of a toilet bowl 803 of the toilet 8. The health monitoring apparatus is used for fitting with the monitoring hole 801 of the toilet 8, such that the monitoring element can extend out to monitor a liquid to be monitored (urine) or the sealing portion 201 of the plugging element 2 can plug the monitoring hole 801. Specifically, a front wall of the toilet 8 is provided with an accommodating chamber 802, the health monitoring apparatus is mounted in the accommodating chamber 802, and the accommodating chamber 802 is in communication with the toilet bowl 803 through the monitoring hole 801.

With reference to FIG. 6 to FIG. 11, the use of the above embodiments may include the following process:

When the health monitoring apparatus is not used, the sealing portion 201 of the plugging element 2 extends out of the accommodating channel 401 to plug the monitoring hole 801;

When user's urine needs to be tested, the transmission unit 3 is operated to drive the plugging element 2 back into the original accommodating channel 401, then the driving unit 5 is operated to drive the container body 4 to move, the initial state that the plugging element 2 corresponds to the monitoring hole 801 (communication) is switched to the state that the monitoring module 1 corresponds to the monitoring hole 801 (communication), the transmission unit 3 is operated to drive the monitoring element (the bracket 101 and the test paper 102) to extend out of the monitoring kit 103, the transmission unit 3 further drives the monitoring element and the monitoring kit 103 together to continue to extend out of the accommodating channel 401, and the monitoring element extends from the monitoring hole 801 into the toilet bowl 803 to receive urine for monitoring;

After the monitoring is completed, the transmission unit 3 is operated to drive the used monitoring element back into the monitoring kit 103, and to further drive the monitoring module 1 back into the original accommodating channel 401; the driving unit 5 is operated to drive the container body 4 to move, the state that the monitoring module 1 corresponds to the monitoring hole 801 (communication) is switched to the state that the plugging element 2 corresponds to the monitoring hole 801, and then the transmission unit 3 is operated to drive the plugging element 2 to extend out of the accommodating channel 401 to plug the monitoring hole 801.

Compared with the prior art, the beneficial effects of this embodiment include at least the following:
1. The health monitoring apparatus provided in this embodiment includes the monitoring module, the plugging element, and the transmission member capable of being in transmission fit with the monitoring module or the plugging element; the monitoring module includes the monitoring element capable of monitoring the liquid to be monitored, and the plugging element has the sealing portion for plugging the monitoring hole of the toilet; during monitoring, the transmission member drives the monitoring module, such that the monitoring element extends out of the monitoring hole for monitoring; when no monitoring is performed, the transmission member drives the plugging element, such that the sealing portion is in sealing fit with the monitoring hole. The same transmission unit, which is in transmission fit with the monitoring module or the plugging element respectively, drives the monitoring module to extend out during monitoring, and drives the plugging element to extend out to seal the monitoring hole when no monitoring is performed. Therefore, the normal use function of the toilet is not affected, the monitoring module is prevented from being contaminated, and the health monitoring apparatus has a simple structure and reliable functions;
2. The health monitoring apparatus provided in this embodiment further includes the container body and the driving unit, where the container body has the plurality of accommodating channels inside, and the driving unit includes the driving member in transmission fit with the container body; the driving member drives the container body to move, such that at least one of the accommodating channels corresponds to the monitoring hole; the monitoring module is arranged in the accommodating channel in an extensible and retractable manner; therefore, the overall structure is compact, and the cost is low. The container body moves to change the position of the accommodating channel, such that the accommodating channel corresponding to the monitoring hole changes with the monitoring module arranged therein in the extensible and retractable manner; therefore, the health monitoring apparatus is skillful, simple, and easy to operate. The fit between the driving mechanism and the transmission mechanism can achieve automatic replacement of the monitoring module, so the health monitoring apparatus is convenient to use and does not require manual replacement;
3. When the liquid to be monitored is monitored, the container body moves until one of the monitoring modules corresponds to the monitoring hole, and the transmission mechanism drives the monitoring module to extend out of the corresponding accommodating channel; after the liquid to be monitored is monitored, the monitoring module retracts into the original accommodating channel; or the monitoring module is discarded, so an additional waste recycling box is not required, product volume is reduced, and the space is saved;
4. In this embodiment, after the monitoring elements are used up, the container body can be replaced with a new one, without the need for manually contacting the test paper stained with urine, thereby further improving user experience and comfort.

### Embodiment 2:

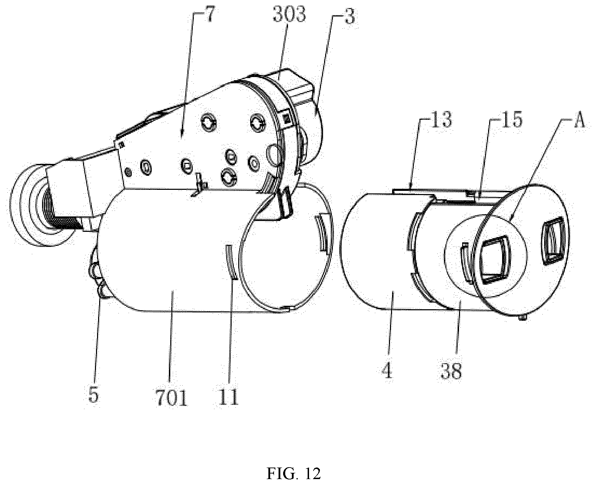

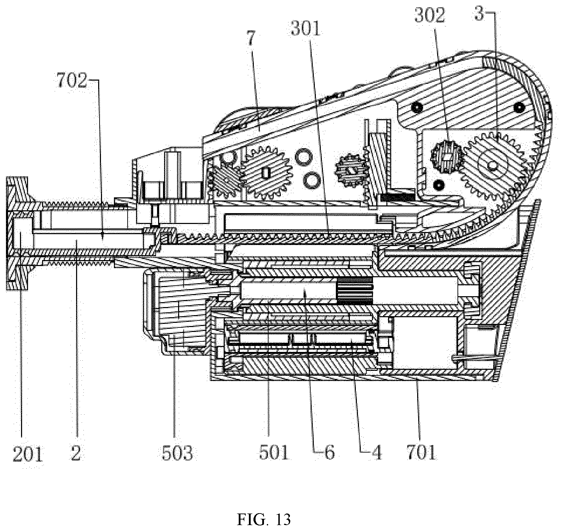

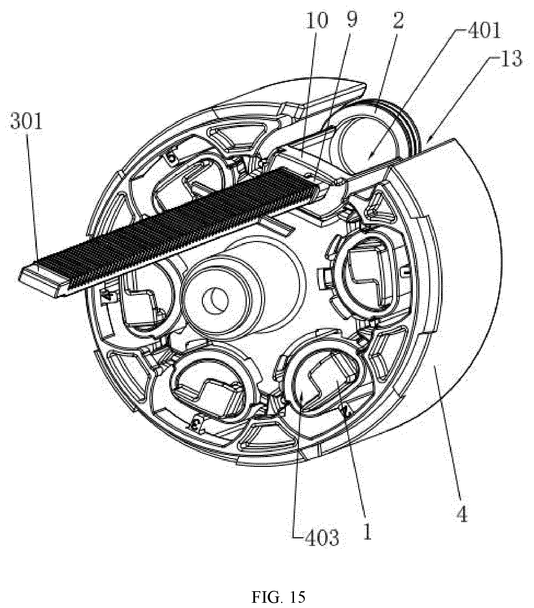

A health monitoring apparatus, as shown in FIGs. 12, 13, 14, and 15, includes a fixing frame 7 mounted in a toilet 8, the fixing frame 7 includes a mounting shell 701, the mounting shell 701 penetrates a rear side wall of the toilet 8, a container body 4 is detachably connected inside the mounting shell 701, the container body 4 is provided with a first accommodating chamber 402 and a plurality of second accommodating chambers 403, a plugging element 2 is mounted in the first accommodating chamber 402, a monitoring module 1 is mounted in each of the plurality of second accommodating chambers 403, the monitoring module 1 includes a bracket 101, and test paper 102 is placed on the bracket 101.

The toilet 8 is provided with a monitoring hole 801 on one side of a main flushing hole, a channel 702 corresponding to the monitoring hole 801 is provided in the fixing frame 7, and the plugging element 2 and the monitoring module 1 can move in the channel 702. The end of the channel 702 near the monitoring hole 801 is open, such that the monitoring module 1 can extend out of the channel 702. When the monitoring module 1 is not used, the plugging element 2 is plugged in the channel 702 to prevent water leakage from the toilet 8. A sealing portion 201 is fixed at the end of the plugging element 2 near the monitoring hole 801, which can improve the sealing property.

A transmission member for driving the plugging element 2 and the monitoring module 1 to move along the channel 702 is provided above the mounting shell 701 in the fixing frame 7, the transmission member includes a first power member 503 mounted in the fixing frame 7, a gear set 302 is connected to an output shaft of the first power member 503, a rack 301 meshing with the gear set 302 is connected inside the fixing frame 7 in a sliding manner, and the first power member 503 drives the rack 301 to move in the fixing frame 7 and the channel 702 through the gear set 302.

When the container body 4 rotates to the first accommodating chamber 402 or the second accommodating chamber 403 and is flush with the channel 702, an end of the rack 301 is detachably connected to the plugging element 2 or the monitoring module 1, thereby driving the plugging element 2 or the monitoring module 1 to move in the channel 702.

A driving unit 5 for driving the container body 4 to rotate is provided on the fixing frame 7, the driving unit 5 specifically includes a first power member 503 mounted on an outer wall of the mounting shell 701, a driving shaft is connected to an output end of the first power member 503, the driving shaft penetrates into the mounting shell 701, an insertion hole 6 in insertion fit with the driving shaft is provided in the container body 4, and the driving shaft is in limiting fit with the insertion hole 6 circumferentially, thereby driving the container body 4 to rotate,

A driving axis of the container body 4 is parallel to a direction of the channel 702, and the first accommodating chamber 402 and the plurality of second accommodating chambers 403 are arranged circumferentially along the driving axis of the container body 4.

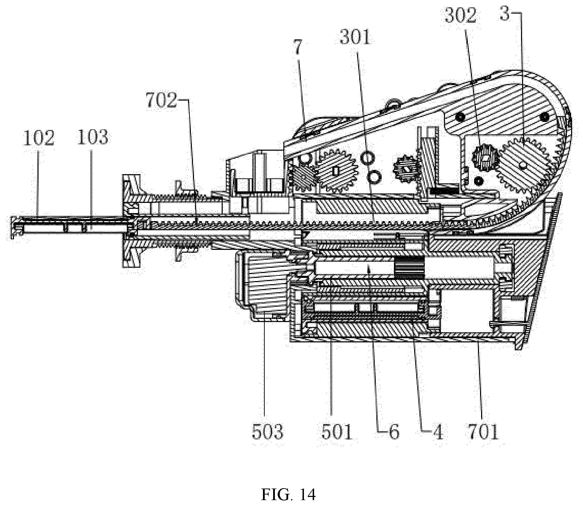

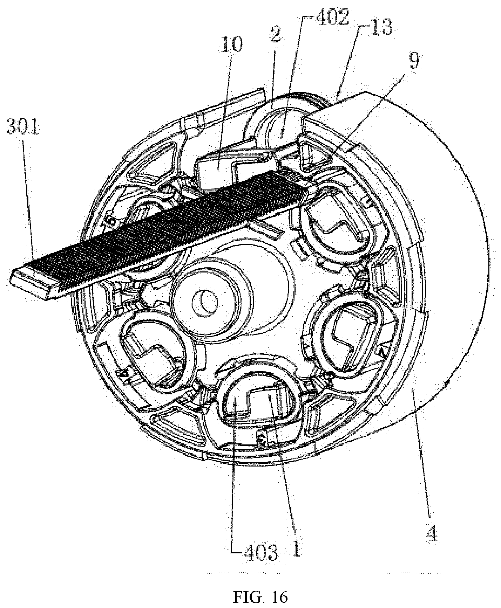

As shown in FIGs. 14, 15, and 16, the end of the rack 301 near the channel 702 is provided with a limit portion 9, and an end of each of the plugging element 2 and the monitoring module 1 is provided with a fit portion 10 for clamping with the limit portion 9; when the container body 4 rotates forward until the plugging element 2 or the monitoring module 1 corresponds to the channel 702, the limit portion 9 is clamped in the fit portion 10 to form limiting fit; when the container body 4 rotates reversely to a middle portion of the adjacent accommodating channel 401, the limit portion 9 rotates out from the fit portion 10, thereby achieving detachable connection between the end of the rack 301 and the plugging element 2 or the monitoring module 1.

When the user needs to use the monitoring module 1, the rack 301 drives the plugging element 2 to retract into the first accommodating chamber 402, and the first power member 503 drives the container body 4 to rotate, such that the limit portion 9 of the rack 301 separates from the fit portion 10 of the plugging element 2, and the plugging element 2 separates from the rack 301. At this time, the rack 301 moves backward a short distance to yield the monitoring module 1. When the monitoring module 1 rotates to an angle at which the plugging element 2 separates from the rack 301, the rack 301 moves forward to its original position. Then, the container body 4 rotates until the monitoring module 1 is parallel to the channel 702, the fit portion 10 on the monitoring module 1 is in limiting fit with the limit portion 9 of the rack 301, and the rack 301 can drive the monitoring module 1 to extend out of the channel 702.

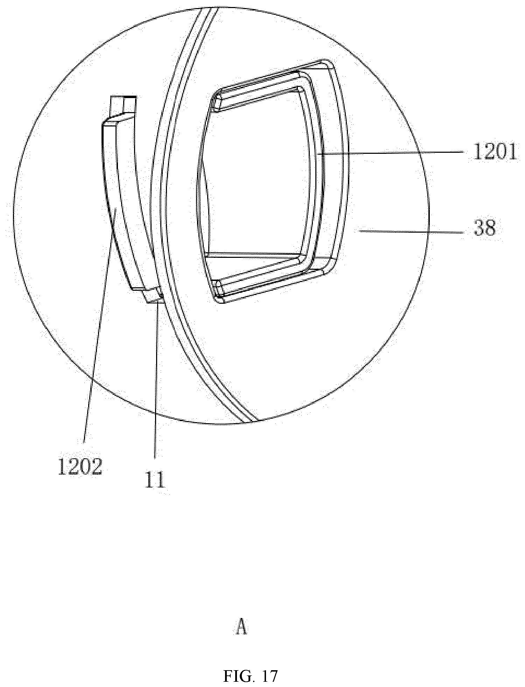

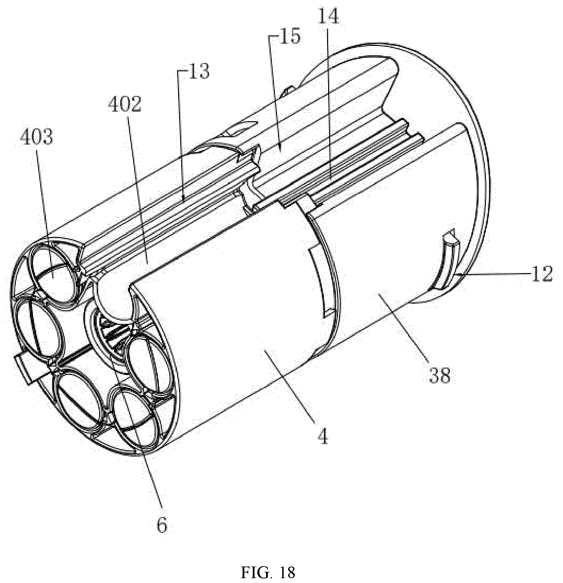

As shown in FIGs. 12, 17, and 18, the health monitoring apparatus further includes a rear cover assembly 38 rotatably connected to the container body 4, and the container body 4 and the rear cover assembly 38 form a container assembly. The mounting shell 701 is provided with a first clamping portion 11. The first clamping portion 11 specifically includes two clamping holes formed on side walls of the mounting shell 701, with one on each of the left and right sides. The rear cover assembly 38 is provided with a second clamping portion 12 in detachable clamping fit with the first clamping portion 11.

The second clamping portion 12 specifically includes two movable slots formed at an end of the rear cover assembly 38. An operation block 1201 is movably provided in the movable slot through a spring, a clamping block 1202 is fixed on the operation block 1201, and the clamping block 1202 penetrates a side wall of the rear cover assembly 38. When the spring is in a natural extension state, the clamping block 1202 penetrates into the first clamping portion 11 to achieve the fixation between the rear cover assembly 38 and the mounting shell 701, and the driving unit 5 drives only the container body 4 to rotate.

The container body 4 is provided with a yielding portion 13 in yielding fit with the rack 301, an upper portion of the first accommodating chamber 402 is a first slot which is open, the first slot forms the yielding portion 13, and the rear cover assembly 38 is provided with a slide 14 that allows the rack 301 to pass through, the slide 14 is flush with the channel 702. An upper portion of the slide 14 is a second slot 15 which is open, such that the rack 301 can pass through the slide 14. When the container assembly is removed, the first slot and the second slot 15 yield the rack 301.

The working process in this embodiment is as follows: In an ordinary state, the plugging element 2 is plugged in the channel 702 to prevent water leakage from the toilet 8. When the user needs to use the monitoring module 1, the rack 301 drives the plugging element 2 to retract into the first accommodating chamber 402, the first power member 503 drives the container assembly to rotate until the second accommodating chamber 403 is flush with the channel 702, and the rack 301 then drives the monitoring module 1 in the second accommodating chamber 403 to extend out from an opening of the channel 702, such that the monitoring module 1 is located in the toilet 8, and a urine test can be performed. After the urine test is completed, the above operation is repeated for the transmission member, and then the plugging element 2 is pushed out and plugged in the channel 702;

When the user needs to replace the monitoring module 1, the user moves around to the back of the toilet 8 and presses the two operation blocks 1201 on the rear cover assembly 38 towards the middle, the clamping blocks 1202 separate from the first clamping portion 11, the container assembly can be removed from the mounting shell 701, the monitoring module 1 in the container body 4 is replaced, and the plugging element 2 is still plugged in the channel 702.

In summary, the monitoring component is convenient to replace in this embodiment; and when the monitoring component is replaced, the plug is still plugged in the channel, so even if the container assembly is not yet mounted back, the toilet can still be used normally without water leakage, and the safety is higher. By configuring the rear cover assembly, the first clamping portion, and the second clamping portion, when the second driving assembly drives the container body to rotate, the entire container assembly is fixed relative to the mounting shell, thereby improving the stability of the container assembly and avoiding detachment.

### Embodiment 3:

This embodiment provides a health monitoring apparatus arranged on a toilet 8.

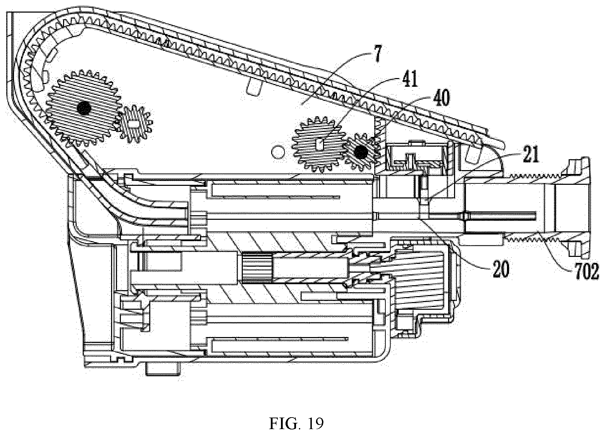

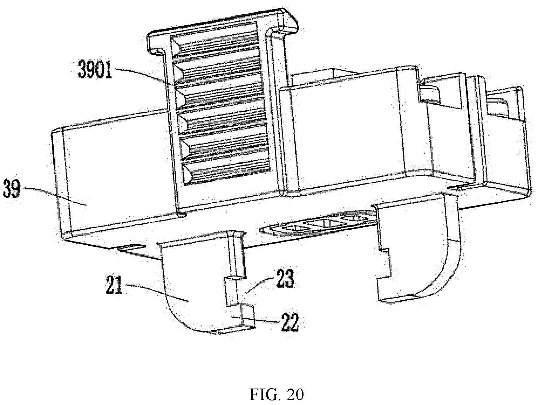

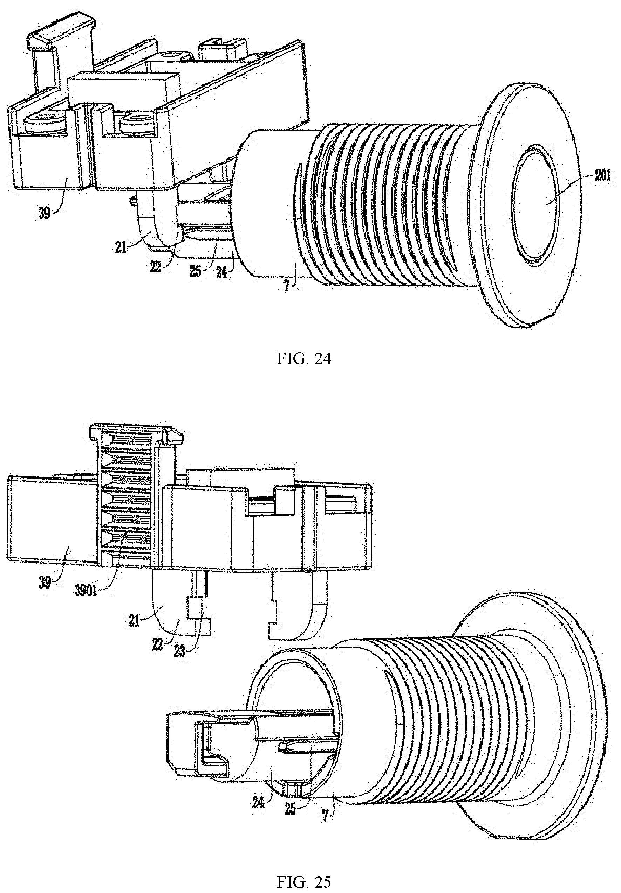

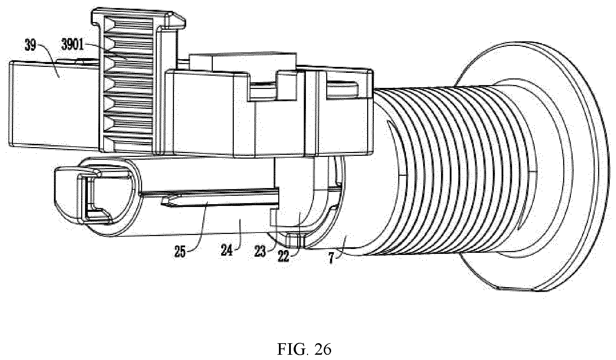

As shown in FIG. 19 to FIG. 26, the health monitoring apparatus includes a fixing frame 7, a movable member 24, and a limit member 21. The movable member 24 and the limit member 21 are arranged on the fixing frame 7. The fixing frame 7 includes a channel 702 for extracting a sample to be monitored. The movable member 24 forms a sealing relationship with the channel 702, and the movable member 24 is movable relative to the channel 702. The limit member 21 includes a limiting state and at least one yielding state, and the limit member 21 is capable of switching between the limiting state and the at least one yielding state.

The movable member 24 forms a sealing fit relationship with the channel 702 on a first position, the movable member 24 includes at least one stop portion 25, the limit member 21 switches to the limiting state, the limit member 21 fits with the at least one stop portion 25, and the movable member 24 is limited at the first position. When the limit member 21 switches to the yielding state, the limit member 21 disengages from the fit with the stop portion 25, and the movable member 24 is released from the limiting.

The movable member 24 is movable on the fixing frame 7 to achieve other functions, and the movable member 24 forms multiple fitting relationships with the channel 702 at multiple different positions. For example, the fitting positions of the movable member 24 and the channel 702 may include a first position, a second position, and a third position. The first position is a position where the movable member 24 plugs the channel 702 when the movable member 24 does not move in the ordinary state. The second position may be a position required for the movable member 24 to transfer the sample to be monitored to a shell 39. The third position may be a position where the movable member 24 extracts the sample to be monitored. Of course, there may be other fitting positions. In the first position, the movable member 24 mainly maintains a stable sealing relationship with the channel 702 to avoid water leakage. Of course, the first position may alternatively be a position for other purposes. For example: the first position may be an initial position where the movable member 24 is located when it is not in operation, or the first position may be a recognition position where the movable member 24 corresponding to a recognition module monitors and analyzes the monitoring element.

The limit member 21 includes a limiting state and at least one yielding state, so the limit member 21 can achieve various forms with the stop portion 25 through different positional relationships, fitting relationships, etc., and can fit with the movable member 24 to move to meet the yielding or limiting requirements at different positions.

In the case where the movable member 24 is movable, at least one stop portion 25 and a limit member 21 capable of switching states are provided on the movable member 24. When the limit member 21 is switched to the limiting state and the movable member 24 is in a sealing position, the limit member 21 and the movable member 24 can achieve a sealing relationship. Therefore, monitoring and extraction functions can be achieved while a monitoring requirement is met, and the problem of water leakage caused by poor sealing of the channel 702 in the monitoring apparatus is avoided when there is no monitoring requirement.

As shown in FIG. 19, in a specific embodiment, the fixing frame 7 may include a housing of the monitoring apparatus, the channel 702 is formed in the housing, and the housing is connected to the inner wall of the channel 702 of the toilet 8 by external threads.

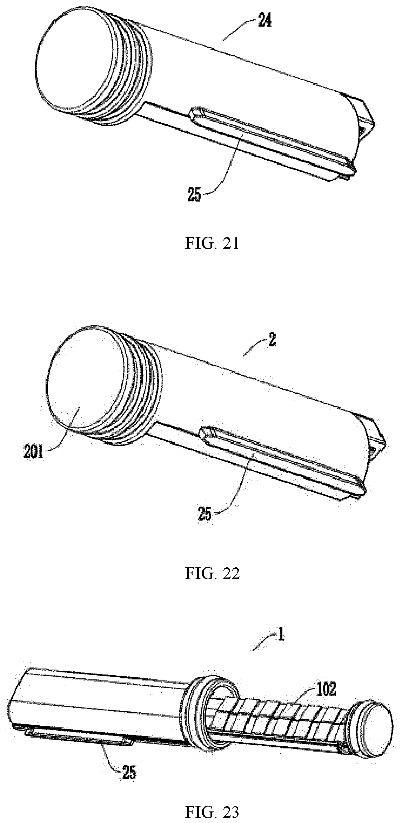

As shown in FIG. 21 to FIG. 23, in this embodiment, the stop portion 25 is a raised structure, and the stop portion 25 is arranged on an outer wall of the movable member 24. The stop portion 25, which is a raised structure and is arranged on the outer wall of the movable member 24, easily fits with the limit member 21, with guaranteed stability. It can be understood that the raised structure may be a cylindrical structure with an axis perpendicular to the direction of movement, a square raised structure, a hemispherical raised structure, or other shaped structures.

As shown in FIG. 19, in this embodiment, the fixing frame 7 is provided with a guide groove 20, and the stop portion 25 is movable along the guide groove 20. In the limiting state, the limit member 21 plugs the guide groove 20, and the stop portion 25 is limited from moving along the guide groove 20 into the fixing frame 7. By providing the guide groove 20 on the fixing frame 7 to guide the movement of the stop portion 25, the limit member 21 can accurately fit with the stop portion 25 at a specific position.

As shown in FIG. 21 to FIG. 23, in some implementations, the movable member 24 may be of a cylindrical structure or a partially cylindrical structure, and the cylindrical structure can form a sealing relationship with the side wall of the channel 702.

Preferably, the stop portion 25 is a raised rib, the stop portion 25 extends in the direction of movement of the movable member 24, the guide groove 20 is a strip groove, and the guide groove 20 extends in the direction of movement of the movable member 24. The stop portion 25 of the rib structure has good mechanical strength. On the other hand, the stop portion can form a fitting relationship with the guide groove 20 at a relatively long distance, thereby avoiding the phenomena such as shaking and deflection of the movable member 24 during movement, and ensuring that the movable member 24 can still maintain a good sealing relationship with the channel 702 during the movement.

As shown in FIGs. 19 and 24-26, in this embodiment, the movable member 24 is movable along an axis of the channel 702, the movable member 24 may be the monitoring module 1, and the monitoring module 1 includes a monitoring kit 103 and a monitoring element. The monitoring module 1 is in the first position, the monitoring kit 103 is in sealing fit with the channel 702, and the monitoring element can extend from the channel 702 into a chamber of the toilet 8 to extract a sample to be monitored. After the sample to be monitored is extracted, the monitoring element retracts from the channel 702 and moves to a place below the shell 39, and a monitoring function is achieved by a recognition structure on the shell 39. The monitoring element has an end surface that can be used for plugging the opening of the channel 702, thereby avoiding the formation of a leaking channel 702.

In this embodiment, during the retraction of the monitoring element to the monitoring kit 103, the limit member 21 switches to the limiting state to limit the monitoring kit 103. After the monitoring element retracts to a preset position, the limit member 21 switches to the yielding state to yield the monitoring kit 103.

The monitoring element and the monitoring kit 103 may be two parts, the monitoring element is arranged in the monitoring kit 103, and the monitoring kit 103 is still in sealing fit with the channel 702. The monitoring element has at least two sealing structures. During the movement of the monitoring element, at least one sealing structure of the monitoring element fits with the monitoring kit 103 to avoid water leakage at the joint between the monitoring element and the monitoring kit 103.

As shown in FIG. 21 to FIG. 24, in this embodiment, the movable member 24 may be a one-piece plugging element 2. The plugging element 2 includes a monitoring kit 103 and an end surface at one end of the monitoring kit 103, the monitoring kit 103 forms a sealing relationship with the channel 702, and the end surface is used for plugging the opening of the channel 702. The plugging element 2 forms a sealing relationship between the monitoring kit 103 and the channel 702, and plugs the opening of the channel 702 through the end surface, thereby avoiding the possibility of water leakage at any position where the plugging element 2 fits with the channel 702. When the plugging element 2 moves axially along the channel 702, the plugging element 2 can continue to form a sealing relationship with the inner wall of the channel 702 through the monitoring kit 103.

In some implementations, the monitoring kit 103 and the end surface of the plugging element 2 are integrally formed. The key sealing portion 201 of the plugging element 2 may be one-piece, which is conducive to improving the sealing effect.

Different members may be used and even switched in different application scenarios. It can be understood that the movable member 24 may be designed as a member compatible with a testing function, and some components are replaced to meet the structural requirements of the plugging element 2 and the monitoring module 1.

In some embodiments, a second rack 3901 may be provided on the shell 39, and a transmission assembly capable of driving the shell 39 is provided inside the monitoring apparatus. For example, the transmission assembly includes a second gear set 40 and a second motor 41, an output end of the second motor 41 is connected to the second gear set 40, and the second gear set 40 meshes with the second rack 3901 to drive the shell 39 to move. Specifically, the shell 39 moves linearly back and forth in a radial direction of the channel 702.

It is understandable that the limit member 21 is preferably arranged on the shell 39. The limit member 21 can switch between different states in other ways. For example, the limit member 21 is arranged on the output end of the second motor 41 or a screw, and the second motor 41 drives the switching of the limit member 21.

In this embodiment, the limit member 21 is of a plate structure, the limit member 21 includes at least one plugging plate 22, and the at least one plugging plate 22 extends in a direction perpendicular to the axial direction of the channel 702. The limit member 21 of the plate structure fits with the stop structure at a large area, which can ensure the formation of the fitting relationship and the stability of the fitting relationship.

In this embodiment, a yielding groove 23 is formed on the plugging plate 22, and when the yielding groove 23 corresponds to the stop portion 25, the stop portion 25 can pass through the yielding groove 23. The limit member 21 includes two yielding states, and the positional relationship between the limit member 21 and the stop portion 25 is different in different yielding states. In the first yielding state, the limit member 21 switches to a position offsetting a channel 702 through which the stop portion 25 passes, so as to yield the stop portion 25. In the second yielding state, the yielding groove 23 corresponds to the stop portion 25.

By forming the yielding groove 23 on the plugging plate 22, a yielding state between the limit member 21 and the stop portion 25 is achieved. The yielding state can be specifically set according to the distance, position, and other requirements between the shell 39 and the movable member 24, to provide a stable positional relationship for the implementation of functions of the shell 39. By setting different positional relationships between the limit member 21 and the stop portion 25, multiple yielding states can be achieved, which can provide different states for the implementation of functions of the monitoring apparatus, with the advantage of convenient state switching.

In a specific embodiment, the limit member 21 includes two symmetrically arranged plugging plates 22, the yielding grooves 23 of the two plugging plates 22 face each other, two corresponding sides of the movable member 24 are provided with stop portions 25 respectively, and one plugging plate 22 fits with one stop portion 25 to form a fit on two sides of the movable member 24 respectively. By configuring the two plugging plates 22, the stop portions 25 can be limited at two symmetrical positions in the limiting state, thereby ensuring the stability of the limiting state. Meanwhile, through the two opposing yielding grooves 23, the movable member 24 moves smoothly between the two yielding grooves 23, thereby avoiding shaking and deflection of the movable member 24 during movement and improving the accuracy of monitoring.

It can be understood that the cross-section of the yielding groove 23 may be semi-circular, V-shaped, U-shaped, T-shaped, etc. Preferably, the yielding groove 23 may be a U-shaped groove, and a top surface and a bottom surface of the yielding groove 23 ensure that force acting on the stop portion 25 is perpendicular to the direction of movement of the movable member 24, which is conducive to the smooth movement of the movable member 24 and reduces the phenomenon of left and right shaking and deflection.

In this embodiment, when the movable member 24 is the monitoring module 1, the relationship between the movable member 24 and the limit member 21 during the movement of the movable member 24 may be as follows:

During the movement of the monitoring module 1 towards the outside of the fixing frame 7, the shell 39 moves to a high position, and the limit member 21 is in the yielding state of offsetting the channel 702 through which the stop portion 25 passes, to yield space for the movement of the monitoring module 1.

After the monitoring module 1 moves in place, when the extraction of a sample to be monitored is prepared, the shell 39 moves downward, the limit member 21 switches to the limiting state, the plugging plate 22 fits with the stop portion 25 to limit the retraction of the monitoring kit 103, and the monitoring element extends to the toilet bowl 803 to contact the sample to be monitored.

During the retraction of the monitoring element to the monitoring kit 103, the limit member 21 is still in the limiting state to limit the monitoring kit 103. After the monitoring element retracts to the preset position, if there is any abnormality, the movable member 24 maintains its current state, the monitoring kit 103 plugs the channel 702, and the limit member 21 fits with the stop portion 25 to plug the stop portion 25.

After the monitoring element is recycled, the limit member 21 switches to the yielding state to yield the monitoring module 1.

During the movement of the monitoring module 1 towards the inside of the fixing frame 7, the shell 39 continues to move downward. At this time, the yielding groove 23 is aligned with the stop portion 25, and the entire movable member 24 can move towards the inside of the fixing frame 7, guided by the fit between the yielding groove 23 and the stop portion 25.

After the guided movement is completed, the stop portion 25 of the movable member 24 has moved to the rear of the limit member 21, and the shell 39 moves upward to yield space for the monitoring module 1 to move towards the fixing frame 7.

In this embodiment, when the movable member 24 is the plugging element 2, the relationship between the movable member 24 and the limit member 21 during the movement of the movable member 24 may be as follows:

During the movement of the plugging element 2 towards the outside of the fixing frame 7, the shell 39 moves to a high position, and the limit member 21 is in the yielding state of offsetting the channel 702 through which the stop portion 25 passes, to yield space for the movement of the plugging element 2.

After the plugging element 2 moves in place, the plugging element 2 is in sealing fit with the channel 702, the shell 39 moves downward, the limit member 21 switches to the limiting state, and the plugging plate 22 fits with the stop portion 25 to limit the retraction of the plugging element 2.

During the movement of the plugging element 2 towards the inside of the fixing frame 7, the shell 39 moves to a high position, and the limit member 21 is in the yielding state of offsetting the channel 702 through which the stop portion 25 passes, to yield space for the movement of the plugging element 2.

In this embodiment of the present invention, an intelligent toilet 8 is provided. The intelligent toilet 8 includes the aforementioned health monitoring apparatus and a toilet 8. The toilet 8 includes a chamber capable of storing a sample to be monitored, the fixing frame 7 is arranged on the toilet 8, and the channel 702 is in communication with the chamber.

In this embodiment, when the health monitoring apparatus is arranged on the toilet 8 and the movable member 24 is movable, at least one stop portion 25 and a limit member 21 capable of switching states are provided on the movable member 24 and can achieve a sealing relationship in specific positions and states. Therefore, on the premise of meeting the monitoring function, the problem of water leakage caused by poor sealing of the channel 702 in the monitoring apparatus is avoided.

### Embodiment 4:

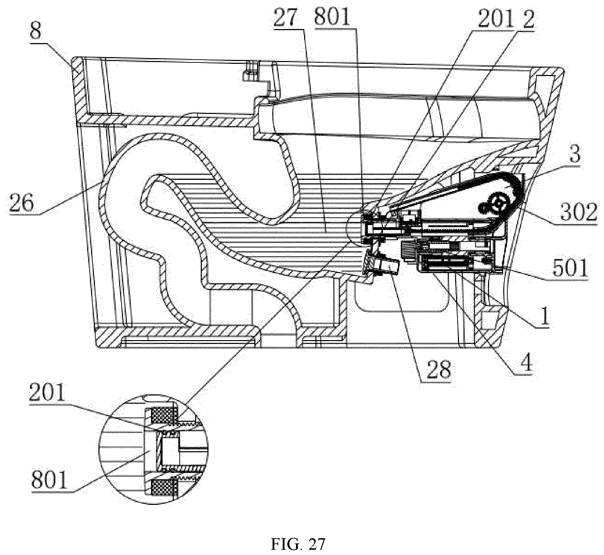

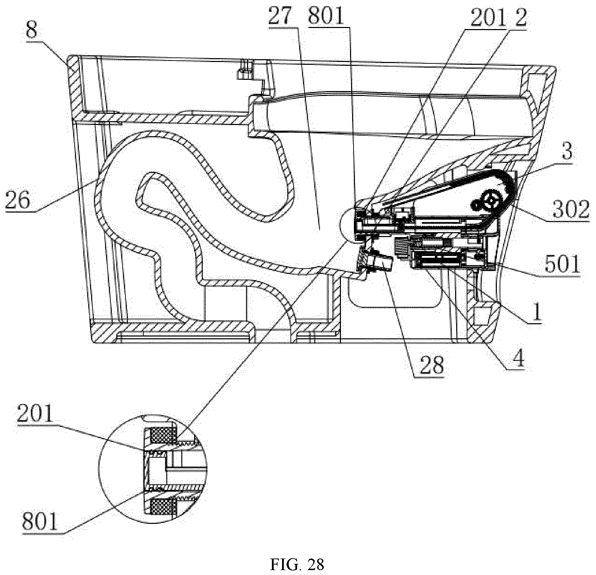

A health monitoring apparatus, as shown in FIG. 27 and FIG. 28, for fitting with a monitoring hole 801 of a toilet 8, includes:
a monitoring module 1 including a monitoring element capable of extending out of the monitoring hole 801 to monitor a liquid to be monitored;
a plugging element 2 with a sealing portion 201 for plugging the monitoring hole 801;
a transmission unit 3 with a driving circuit and a transmission member, where the driving circuit is used for controlling the transmission member to drive the plugging element 2; and
a main control unit capable of monitoring positioning duration of the sealing portion 201 positioned in the monitoring hole 801;

The main control unit is electrically connected to the driving circuit; when the main control unit monitors that the positioning duration exceeds preset duration, the driving circuit controls the transmission member to drive the plugging element 2 to move a preset distance.

In this embodiment, the driving circuit controls the transmission member to drive the plugging element 2 to move the preset distance and then stay at the current position, or the driving circuit controls the transmission member to drive the plugging element 2 to move the preset distance and then reset to its initial position. The driving circuit may be arranged on a main control board or independently arranged from the main control board, as long as the driving circuit is electrically connected to the main control board.

In this embodiment, during the movement of the plugging element 2 by the preset distance, the sealing portion 201 of the plugging element 2 maintains sealing fit with the monitoring hole 801.

In other embodiments, after the plugging element 2 moves the preset distance, the sealing portion 201 separates from the monitoring hole 801.

In this embodiment, the toilet 8 further includes a drain pipe 26 and a water supply unit, where the water supply unit is electrically connected to the main control board. When the positioning duration exceeds the preset duration and before the transmission member drives the plugging element 2 to move, the main control board controls the water supply unit to supply water to the drain pipe 26, such that the drain pipe 26 produces siphon to suck water from a water seal 27 of the toilet 8, and the water in the water seal 27 is lower than a lowest position of the monitoring hole 801, thereby preventing the water in the water seal 27 from entering the monitoring hole 801 to moisturize the monitoring element and affect a monitoring result. Specifically, the water supply unit includes an electromagnetic valve or a water inlet valve and a nozzle 28 arranged at a bottom of the toilet 8. Water is supplied to the water seal 27 through the nozzle 28, and then the drain pipe 26 produces siphon. When the toilet 8 is in normal use, water is also supplied to the water seal 27 through the nozzle 28, and dirt in the water seal 27 is pumped away through the drain pipe 26.

In this embodiment, the health monitoring apparatus further includes a container body 4 and a driving member 501, the container body 4 has a plurality of accommodating channels 401 inside, the monitoring module 1 and the plugging element 2 are arranged in each accommodating channel 401 respectively, and the driving member 501 can drive the container body 4 to move, such that each accommodating channel 401 is in communication with the monitoring hole 801.

In this embodiment, when the container body 4 drives the monitoring module 1 to move to correspond to the monitoring hole 801, the transmission member is in transmission fit with the monitoring module 1; when the container body 4 drives the plugging element 2 to move to correspond to the monitoring hole 801, the transmission member is in transmission fit with the plugging element 2.

In this embodiment, the container body 4 is rotatably arranged, the container body 4 is cylindrical, the accommodating channels 401 penetrate along an axis of the container body 4, and the accommodating channels 401 are arranged sequentially along a circumference of the container body 4.

In this embodiment, the driving member 501 is a driving shaft, a middle portion of the container body 4 is provided with an insertion hole 6, and the driving shaft is in limiting fit with the insertion hole 6 circumferentially.

In this embodiment, one of the driving shaft and the insertion hole 6 is provided with limit ribs 601, the other of the driving shaft and the insertion hole 6 is provided with limit grooves 502, and the limit ribs 601 are adapted to the limit grooves 502.

In this embodiment, the transmission unit 3 further includes a driving motor 303 electrically connected to the driving circuit, and the driving circuit drives the transmission member through the driving motor 303. Specifically, the transmission unit 3 further includes a gear set 302, the transmission member is a rack 301 meshing with the gear set 302, and the driving motor 303 drives the transmission member to move through the gear set 302.

In this embodiment, the main control unit includes a main control board capable of monitoring the positioning duration of the sealing portion 201 positioned in the monitoring hole 801. For example, the main control board calculates the number of steps of the driving motor 303 through a program to deduce the position of the plugging element 2, as long as it can monitor the position of the sealing portion 201 and record the positioning duration of the sealing portion 201.

In other embodiments, the main control unit includes a main control board, as well as a sensing circuit and a timing circuit electrically connected to the main control board respectively, the sensing circuit is used for monitoring the position of the sealing portion 201, and the timing circuit is used for recording the positioning duration of the sealing portion 201. The sensing circuit and the timing circuit are commonly used electrical components in the market, such as a sensing probe and a timer.

In this embodiment, the preset duration is less than 2 days, and the preset distance is less than 4 mm.

A specific working process of this embodiment is as follows:
When the main control board monitors that the positioning duration of the sealing portion 201 positioned in the monitoring hole 801 exceeds 1-2 days, the driving circuit is turned on to control the driving motor 303 to start. The driving motor 303 controls the gear set 302 to drive the transmission member to move, so as to push the plugging element 2 to move by 1 mm to 4 mm. Then, the driving motor 303 controls the gear set 302 to drive the transmission member to reset, so as to drive the plugging element 2 to reset.

In this solution, the main control unit monitors the positioning duration of the sealing portion 201 positioned in the monitoring hole 801, which can periodically drive the plugging element 2 to move, thereby solving the problem of increased starting friction caused by long-term disuse of the plugging element 2, and reducing starting friction, with a simple structure, low cost and high lifespan.

### Embodiment 5:

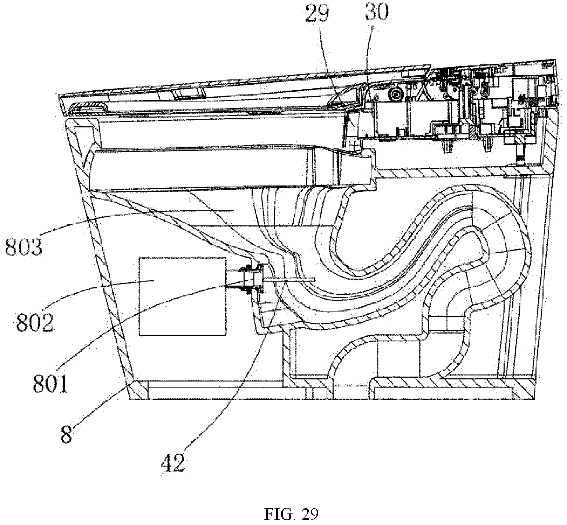

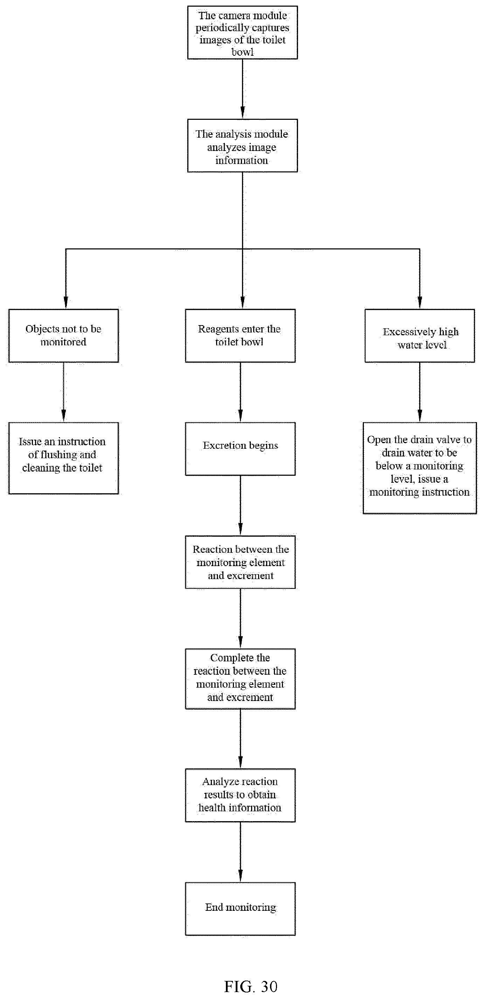

The present invention further provides a health monitoring toilet, as shown in FIG. 29 and FIG. 30, including: a toilet 8, where the toilet 8 has a toilet bowl 803, and a bottom of the toilet bowl 803 is provided with a monitoring hole 801; a monitoring element penetrating into the toilet bowl 803 from the monitoring hole 801 to monitor excrement, which is urine or feces, or a mixture of urine and feces; a camera module 29, where the camera module 29 faces the toilet bowl 803 for capturing; and an analysis module connected to the camera module 29, where information collected by the camera module 29 is transmitted to the analysis module for processing and analysis. The monitoring element extends out from the monitoring hole 801 to monitor the excrement in the toilet bowl 803, and the camera module 29 captures a monitoring result of the monitoring element and transmits the monitoring result to the analysis module for processing and analysis.

In this embodiment, the camera module 29 is connected to the analysis module by a data cable or in a wireless manner.

The health monitoring toilet further includes a base 30 located at an upper portion of the toilet 8, and the camera module 29 is mounted on the base 30.

The camera module 29 includes a hybrid lens, an image sensor, and a filter. The image sensor converts light signals into electrical signals and transmits the electrical signals to the analysis module, and the analysis module analyzes the transmitted signals to obtain a human health status.

The camera module 29 includes a voice coil motor and a lens. The position of the lens is changed by current entering the voice coil motor, to achieve automatic focusing, making the captured images clearer and reducing the errors of analysis on blurry images by the analysis module.

The camera module 29 further includes a lighting source that illuminates at least the monitoring element. The lighting source enhances light signals of the image sensor to improve the intensity, stability, and uniformity of the light signals. Specifically, the lighting source is an LED light. The LED light illuminates the monitoring element and the surrounding environment. During capturing, the results of monitoring by the monitoring element are illuminated to make images clearer, thereby improving the clarity of the images and reducing errors in analysis.

A bottom of a seat of the toilet 8 is further provided with an accommodating chamber 802, the health monitoring apparatus 42 is arranged in the accommodating chamber 802, and the accommodating chamber 802 is in communication with the monitoring hole 801; the health monitoring apparatus 42 includes a monitoring element, the monitoring element includes a bracket 101 and test paper 102, the test paper 102 is mounted on the bracket 101, and reagent blocks are provided on the test paper 102; the reagent blocks react with excrement.

A transmission unit 3 and test paper 102 for monitoring are further provided in the accommodating chamber 802. The transmission unit 3 allows the bracket 101 to carry the test paper 102 from the monitoring hole 801 into the toilet bowl 803 to monitor excrement. The transmission unit 3 can achieve automatic replacement of the test paper 102. Specifically, the transmission unit 3 may be assembled from components such as a motor and a micro cylinder, to achieve automatic replacement of the test paper 102 and allow the test paper 102 to penetrate into the toilet bowl 803 through the monitoring hole 801 for monitoring.

In this embodiment, the camera module 29 faces the toilet bowl 803 for periodical capturing.

In this embodiment, the camera module 29 faces the toilet bowl 803 for capturing, and the analysis module determines whether the monitoring element extends out.

In this embodiment, the camera module 29 faces the toilet bowl 803 for capturing, and the analysis module determines whether there is a foreign object in the toilet bowl 803 or whether there is excrement on the monitoring element; when the foreign object is monitored, a flushing system is controlled to flush; when excrement is monitored on the monitoring element, the camera module 29 captures reaction structures of the monitoring element and the excrement after a preset time and transmits signals to the analysis module to obtain health information, and the health information output from the analysis module is displayed by a device that can display the results, such as a mobile phone or a computer.

In this embodiment, the camera module 29 faces the toilet bowl 803 for periodical capturing, and the analysis module determines a liquid level of the toilet bowl 803; during monitoring, if the liquid level is lower than a threshold, the monitoring element penetrates into the toilet bowl 803 from the monitoring hole 801 to initiate monitoring; during monitoring, if the liquid level is higher than the threshold, the monitoring hole 801 is closed or the toilet bowl 803 is controlled to drain water to reduce the liquid level of the toilet bowl 803.

A working process of this embodiment is as follows: With reference to FIG. 30, the camera module 29 periodically captures images of the toilet bowl 803, that is, once a period of time, and transmits the captured images to the analysis module; during monitoring, the transmission unit 3 drives the monitoring element to penetrate into the toilet bowl 803 through the monitoring hole 801, an excretion instruction prompt is issued for excretion, the excrement reacts with the monitoring element, and after the reaction is completed, the images captured by the camera module 29 are transmitted to the analysis module, which then obtains human health information. At this time, the monitoring ends;

During monitoring, if the camera module 29 monitors that the liquid level in the toilet bowl 803 is higher than the monitoring height, the drain valve is opened to reduce the liquid level to be below the monitoring level; when it is determined that there is a foreign object in the toilet bowl 803, a toilet 8 flushing instruction is activated to flush away the foreign object.

Compared with the prior art, the beneficial effects of this embodiment include at least the following:
1. The camera module is used for capturing towards the toilet bowl and transmitting the monitoring result to the analysis module for data processing and analysis to obtain the human health status, thereby achieving non-contact automatic monitoring, solving the contamination of existing excrement on a device, and better maintaining the hygiene of the device;
2. The position of the lens of the camera module can be changed by the voice coil motor in the camera module, to achieve automatic focusing, making the captured images clearer and reducing errors of analysis on blurry images by the analysis module;
3. By configuring the lighting source, the results of monitoring by the monitoring element are clearer during capturing, and the clarity of images is improved.

### Embodiment 6:

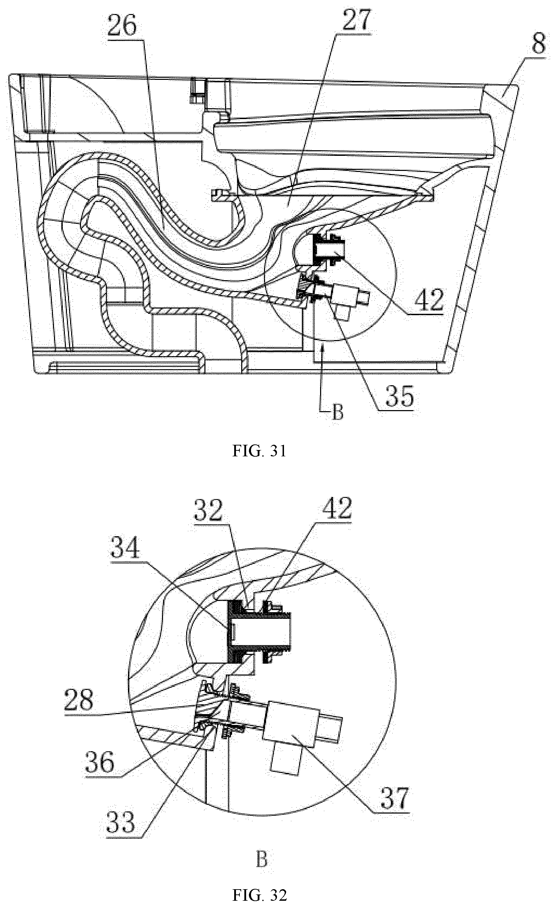

The present invention further provides a health monitoring toilet 8, as shown in FIG. 31 and FIG. 32, including:
a toilet 8 including a toilet bowl 803 and a drain pipe 26, where the toilet bowl 803 has a water seal 27, and water in the water seal 27 can be discharged to the drain pipe 26;
a health monitoring apparatus 42 including a monitoring module 1 for monitoring a sample to be monitored;
a water level sensor 34 for monitoring a water level of the water seal 27;
a spray unit 35 including a nozzle 28 and a control valve 37, where the nozzle 28 has a spray channel 36 for supplying water to the water seal 27, and the control valve 37 is used for controlling the on/off of the spray channel 36;
the drain pipe 26, where the water in the water seal 27 can be discharged to the drain pipe 26; and
a control unit electrically connected to the water level sensor 34 and the health monitoring apparatus 42, where the control unit can control the health monitoring apparatus 42 to turn on based on a monitoring signal of the water level sensor 34.

In this embodiment, the monitoring signal includes a first water level signal, the first water level signal is a signal monitored by the water level sensor 34 that the water level of the water seal 27 is lower than a first height, and the control unit controls the health monitoring apparatus 42 to turn on based on the first water level signal.

In this embodiment, the control unit is further electrically connected to the control valve 37, and the control unit controls the control valve 37 to open upon receiving the first water level signal and after the health monitoring apparatus 42 is turned off.

In this embodiment, the control unit controls the control valve 37 to open and then close after a delay.

In this embodiment, the bottom of the toilet bowl 803 is provided with a first mounting hole 32, and the health monitoring apparatus 42 is mounted in the first mounting hole 32.

In this embodiment, the nozzle 28 is mounted in the first mounting hole 32, or the bottom of the toilet bowl 803 is further provided with a second mounting hole 33, and the nozzle 28 is mounted in the second mounting hole 33. The latter is used in this embodiment.

In this embodiment, the water level sensor 34 is mounted on the health monitoring apparatus 42, or the water level sensor 34 is mounted on the nozzle 28.

In this embodiment, the spray channel 36 sprays water to the water seal 27, such that the drain pipe 26 forms a siphon to suck water from the water seal 27.

In this embodiment, the health monitoring apparatus 42 is a urine test unit or a stool test unit; the control valve 37 is an electromagnetic valve.

In this embodiment, the water level sensor 34 is a capacitive sensor, an electrode needle, or a float switch, and is specifically the capacitive sensor mounted on the health monitoring apparatus 42.

A specific working process of this embodiment is as follows:
During monitoring, the electromagnetic valve is first controlled to open, the nozzle 28 supplies water to the water seal 27 through the spray channel 36, the drain pipe 26 forms a siphon, and the drain pipe 26 sucks water from the water seal 27 through the siphon effect to empty the water seal 27; the water level sensor 34 determines through changes in capacitance whether the water level is below the first height; when the water level is below the first height, the control unit controls the health monitoring apparatus 42 to turn on for monitoring;

After monitoring, the water level sensor 34 determines through changes in capacitance whether the water level exceeds the first height; when the water level is lower than the first height, the control unit controls the electromagnetic valve to open to supply water to the water seal 27; when the water level of the water seal 27 is higher than the first height and the electromagnetic valve is closed after a preset time of water supply, the water seal 27 is replenished.

The beneficial effects of this embodiment include at least the following:
1. This embodiment enables a user to monitor excrement at home simply, reduces the dilution of excrement by water in a water seal area, can ensure the stability and accuracy of monitoring results, and effectively improves the monitoring efficiency and effectiveness.
2. This embodiment involves only one sensor, which can determine whether to turn on the monitoring unit for monitoring and whether to open the electromagnetic valve for water supply, thereby saving costs.

### Embodiment 7:

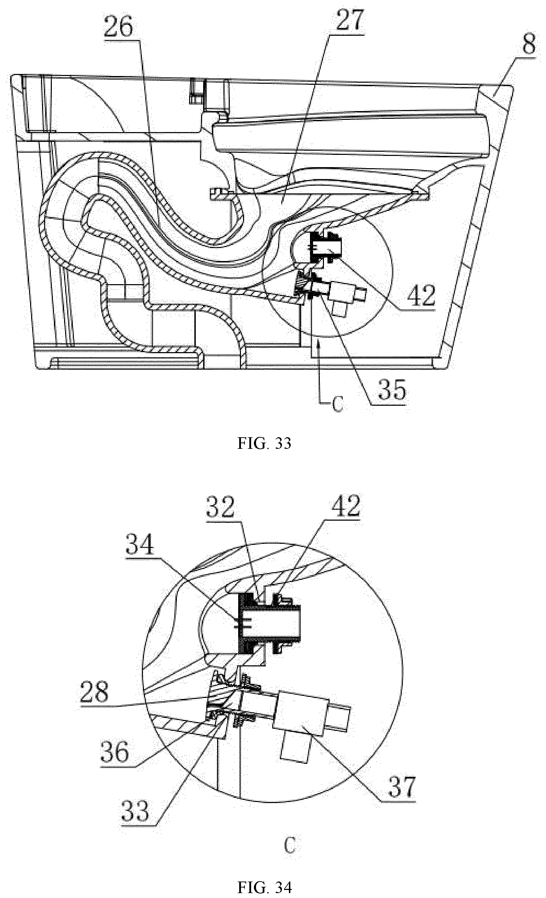

As shown in FIG. 33 and FIG. 34, this embodiment mainly differs from Embodiment 6 in that an electrode needle mounted on the health monitoring apparatus 42 is used in this embodiment.

Other unspecified parts are consistent with Embodiment 6 and will not be repeated here.

### Embodiment 8:

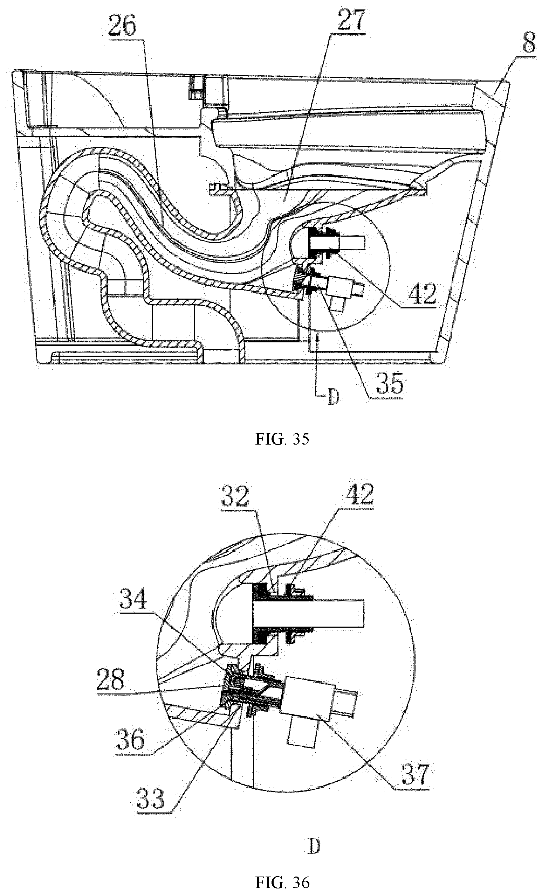

As shown in FIG. 35 and FIG. 36, this embodiment mainly differs from Embodiment 6 in that an electrode needle mounted on the nozzle 28 is used in this embodiment.

Other unspecified parts are consistent with Embodiment 6 and will not be repeated here.

The above descriptions are merely preferred embodiments of the present invention, and are not intended to limit the present invention. Any modification, equivalent substitution, improvement, and the like made within the design concept of the present invention shall fall within the protection scope of the present invention.

## Claims

1. A health monitoring apparatus for fitting with a monitoring hole of a toilet, comprising:
a monitoring module comprising a monitoring element capable of monitoring a liquid to be monitored;
a plugging element with a sealing portion for plugging the monitoring hole; and
a transmission unit with a transmission member capable of being in transmission fit with the monitoring module or the plugging element, wherein during monitoring, the transmission member drives the monitoring module, such that the monitoring element extends out of the monitoring hole for monitoring; when no monitoring is performed, the transmission member drives the plugging element, such that the sealing portion is in sealing fit with the monitoring hole.

2. The health monitoring apparatus according to claim 1, further comprising a container body and a driving unit, wherein the container body has a plurality of accommodating channels inside, and the driving unit comprises a driving member in transmission fit with the container body; the driving member drives the container body to move, such that at least one of the accommodating channels corresponds to the monitoring hole; the monitoring module is arranged in the accommodating channel in an extensible and retractable manner.

3. The health monitoring apparatus according to claim 2, wherein the plugging element is arranged in the accommodating channel in an extensible and retractable manner, or the plugging element is independently arranged outside the container body.

4. The health monitoring apparatus according to claim 2, wherein the container body is rotatably arranged, the container body is cylindrical, the accommodating channels penetrate along an axis of the container body, and the accommodating channels are arranged sequentially along a circumference of the container body.

5. The health monitoring apparatus according to claim 4, wherein the driving member is a driving shaft, a middle portion of the container body is provided with an insertion hole, and the driving shaft is in limiting fit with the insertion hole circumferentially; one of the driving shaft and the insertion hole is provided with limit ribs, the other of the driving shaft and the insertion hole is provided with limit grooves, and the limit ribs are adapted to the limit grooves.

6. The health monitoring apparatus according to claim 2, wherein the container body is arranged in a sliding manner, and the plurality of accommodating channels are arranged sequentially in a sliding direction of the container body.

7. The health monitoring apparatus according to claim 2, wherein the driving unit further comprises a first power member, and the first power member drives the driving member to move.

8. The health monitoring apparatus according to claim 2, further comprising a fixing frame, wherein the container body is detachably mounted on the fixing frame.

9. The health monitoring apparatus according to claim 1, wherein the transmission member is a rack, and the transmission unit further comprises a gear set meshing with the rack and a driving motor driving the gear set.

10. The health monitoring apparatus according to claim 1, wherein the monitoring module further comprises a monitoring kit, the monitoring element is arranged in the monitoring kit in an extensible and retractable manner, and the monitoring element comprises a bracket and test paper laid on the bracket.

11. A health monitoring apparatus, comprising a fixing frame, a plugging element, and a monitoring module, wherein the fixing frame comprises a mounting shell, a container body is detachably connected inside the mounting shell, the container body is provided with a first accommodating chamber for accommodating the plugging element and at least one second accommodating chamber for accommodating the monitoring module, the fixing frame is further provided with a channel corresponding to a monitoring hole of a toilet, the end of the channel near the monitoring hole is open, a transmission unit for driving the plugging element and the monitoring module to move is further provided on the fixing frame, a driving unit for driving the container body to move is further provided on the fixing frame, and the container body is further provided with a yielding portion in yielding fit with the transmission unit; when the container body moves to the first accommodating chamber to correspond to the channel, the transmission unit can drive the plugging element to be limited in the channel, and the container body is in yielding fit with the transmission unit through the yielding portion, such that the container body can be separated from the mounting shell.

12. The health monitoring apparatus according to claim 11, wherein the transmission unit comprises a driving motor mounted on the fixing frame, an output shaft of the driving motor is connected to a gear set, a rack meshing with the gear is provided on the fixing frame in a sliding manner, and the rack is capable of being in transmission fit with the plugging element or the monitoring module.

13. The health monitoring apparatus according to claim 11, wherein the transmission unit is provided with a limit portion, and an end of each of the plugging element and the monitoring module is provided with a fit portion for clamping with the limit portion; when the container body rotates forward until the plugging element or the monitoring module corresponds to the channel, the limit portion is clamped in the fit portion to form limiting fit; when the container body rotates reversely, the limit portion exits from the fit portion, to achieve detachable connection between the transmission unit and the plugging element or the monitoring module.

14. The health monitoring apparatus according to claim 11, further comprising a rear cover assembly rotatably connected to the container body, wherein the container body and the rear cover assembly form a container assembly, the mounting shell is provided with a first clamping portion, the rear cover assembly is provided with a second clamping portion in detachable clamping fit with the first clamping portion, and the driving unit drives the container body to rotate relative to the rear cover assembly.

15. The health monitoring apparatus according to claim 14, wherein an upper portion of the first accommodating chamber is a first slot which is open, the first slot forms the yielding portion, the rear cover assembly is provided with a slide that allows the rack to pass through, and an upper portion of the slide is a second slot which is open; when the container assembly separates from the mounting shell, the first slot and the second slot yield the transmission unit.

16. The health monitoring apparatus according to claim 11, wherein the container body is rotatably arranged, a driving axis of the container body is parallel to a direction of the channel, and the first accommodating chamber and the plurality of second accommodating chambers are arranged along a circumference of the container body.

17. The health monitoring apparatus according to claim 16, wherein the driving unit comprises a first power member mounted on the mounting shell and a driving member arranged at an output end of the first power member; the container body is provided with an insertion hole in insertion fit with the driving member, and the driving member is in limiting fit with the insertion hole circumferentially, thereby driving the container body to rotate.

18. The health monitoring apparatus according to claim 11, wherein the end of the plugging element near the monitoring hole has a sealing portion, and the monitoring module is provided with test paper.

19. A health monitoring apparatus, arranged on a toilet, comprising:
a fixing frame arranged on the toilet, the fixing frame comprising a channel for extracting a sample to be monitored;
a movable member, the movable member being movable relative to the channel; and
a limit member arranged on the fixing frame, the limit member comprising a limiting state and at least one yielding state, and the limit member being capable of switching between the limiting state and the at least one yielding state;
wherein the movable member comprises at least one stop portion, the limit member switches to the limiting state, the limit member fits with the at least one stop portion, and the movable member is limited at a first position; when the limit member switches to the yielding state, the limit member disengages from the fit with the stop portion, and the movable member is released from the limiting.

20. The health monitoring apparatus according to claim 19, wherein the movable member forms a sealing fit relationship with the channel at the first position.

21. The health monitoring apparatus according to claim 19, wherein the movable member can linearly reciprocate in an axial direction of the channel, the stop portion is a protruding rib, and the stop portion extends in a direction of movement of the movable member.

22. The health monitoring apparatus according to claim 19, wherein the fixing frame is provided with a guide groove, and the stop portion is movable along the guide groove; in the limiting state, the limit member plugs the guide groove to limit the stop portion.

23. The health monitoring apparatus according to claim 19, wherein the movable member is a plugging element, and when the movable member is in the first position, the plugging element plugs the channel.

24. The health monitoring apparatus according to claim 19, wherein the movable member is a monitoring module, and the monitoring module comprises a monitoring kit and a monitoring element; when the movable member is in the first position, the monitoring kit forms a sealing fit relationship with the channel, and the monitoring element can extend from the monitoring kit into a chamber of the toilet via the channel to extract the sample to be monitored.

25. The health monitoring apparatus according to claim 24, wherein in the process of retraction of the monitoring element to the monitoring kit, the limit member switches to the limiting state to limit the monitoring kit; after the monitoring element retracts to a preset position, the limit member switches to the yielding state to yield the monitoring kit.

26. The health monitoring apparatus according to claim 19, wherein when the limit member moves to a position offsetting a channel through which the stop portion passes, the limit member is in a first yielding state.

27. The health monitoring apparatus according to claim 19, wherein the limit member is of a plate structure, the limit member comprises at least one plugging plate, and the at least one plugging plate extends in a direction perpendicular to the axial direction of the channel.

28. The health monitoring apparatus according to claim 26, wherein a yielding groove is formed on the at least one plugging plate; when the yielding groove corresponds to the stop portion, the stop portion can pass through the yielding groove, and the limit member is in a second yielding state.

29. A health monitoring apparatus for fitting with a monitoring hole of a toilet, comprising:
a monitoring module comprising a monitoring element capable of extending out of the monitoring hole to monitor a liquid to be monitored;
a plugging element with a sealing portion for plugging the monitoring hole; and
a transmission unit comprising a driving circuit and a transmission member, wherein the driving circuit is used for controlling the transmission member to drive the plugging element; and
a main control unit capable of monitoring positioning duration of the sealing portion in the monitoring hole, wherein the main control unit is electrically connected to the driving circuit; when the main control unit monitors that the positioning duration exceeds preset duration, the driving circuit controls the transmission member to drive the plugging element to move a preset distance.

30. The health monitoring apparatus according to claim 29, wherein in the process that the plugging element moves the preset distance, the sealing portion of the plugging element maintains a sealing fit with the monitoring hole; or after the plugging element moves the preset distance, the sealing portion is separated from the monitoring hole.

31. The health monitoring apparatus according to claim 29, further comprising a container body and a driving member, wherein the container body has a plurality of accommodating channels inside, the monitoring module and the plugging element are arranged in each accommodating channel respectively, and the driving member can drive the container body to move, such that each accommodating channel is in communication with the monitoring hole.

32. The health monitoring apparatus according to claim 31, wherein when the container body drives the monitoring module to move to correspond to the monitoring hole, the transmission member is in transmission fit with the monitoring module; when the container body drives the plugging element to move to correspond to the monitoring hole, the transmission member is in transmission fit with the plugging element.

33. The health monitoring apparatus according to claim 31, wherein the container body is rotatably arranged, the container body is cylindrical, the accommodating channels penetrate along an axis of the container body, and the accommodating channels are arranged sequentially along a circumference of the container body; the driving member is a driving shaft, a middle portion of the container body is provided with an insertion hole, and the driving shaft is in limiting fit with the insertion hole circumferentially.

34. The health monitoring apparatus according to claim 29, wherein the main control unit comprises a main control board, and the main control board can monitor the positioning duration of the sealing portion positioned in the monitoring hole, or
the main control unit comprises a main control board, as well as a sensing circuit and a timing circuit electrically connected to the main control board respectively, the sensing circuit is used for monitoring the position of the sealing portion, and the timing circuit is used for recording the positioning duration of the sealing portion.

35. The health monitoring apparatus according to claim 29, wherein the preset duration is less than 2 days and the preset distance is less than 4 mm.

36. A health monitoring toilet, comprising:
a toilet having a toilet bowl, a bottom of which is provided with a monitoring hole;
a monitoring element penetrating into the toilet bowl from the monitoring hole to monitor excrement;
a camera module for capturing towards the toilet bowl; and
an analysis module connected to the camera module, wherein information collected by the camera module is transmitted to the analysis module for processing and analysis;
wherein the monitoring element extends out from the monitoring hole to monitor excrement in the toilet bowel, and the camera module captures a monitoring result of the monitoring element and transmits the monitoring result to the analysis module for processing and analysis.

37. The health monitoring toilet according to claim 36, further comprising a base located at an upper portion of the toilet, wherein the camera module is mounted on the base.

38. The health monitoring toilet according to claim 36, wherein the camera module comprises an image sensor, and the image sensor converts light signals into electrical signals and transmits the electrical signals to the analysis module.

39. The health monitoring toilet according to claim 36, wherein the camera module comprises a voice coil motor and a lens, and the position of the lens is changed by current entering the voice coil motor to achieve automatic focusing.

40. The health monitoring toilet according to claim 36, wherein the camera module further comprises a lighting source that illuminates at least the monitoring element, and the lighting source enhances the light signals of the image sensor.

41. The health monitoring toilet according to claim 36, wherein a bottom of the toilet is further provided with an accommodating chamber, the monitoring element is arranged in the accommodating chamber, and the accommodating chamber is in communication with the monitoring hole; the monitoring element comprises a bracket and test paper, the test paper is mounted on the bracket, and reagent blocks are provided on the test paper.

42. The health monitoring toilet according to claim 36, wherein the camera module faces the toilet bowl for periodical capturing.

43. The health monitoring toilet according to claim 36, wherein the camera module faces the toilet bowl for capturing, and the analysis module determines whether the monitoring element extends out.

44. The health monitoring toilet according to claim 36, wherein the camera module faces the toilet bowl for capturing, and the analysis module determines whether there is a foreign object in the toilet bowl or whether there is excrement on the monitoring element; when the foreign object is monitored, a flushing system is controlled to flush; when excrement is monitored on the monitoring element, the camera module captures reaction structures of the monitoring element and the excrement after a preset time and transmits signals to the analysis module to obtain health information.

45. The health monitoring toilet according to claim 36, wherein the camera module faces the toilet bowl for capturing, and the analysis module determines a liquid level of the toilet bowl; during monitoring, if the liquid level is lower than a threshold, the monitoring element penetrates into the toilet bowl from the monitoring hole to initiate monitoring; during monitoring, if the liquid level is higher than the threshold, the monitoring hole is closed or the toilet bowl is controlled to drain water to reduce the liquid level of the toilet bowl.

46. A health monitoring toilet, comprising:
a toilet comprising a toilet bowl and a drain pipe, wherein the toilet bowl has a water seal, and water in the water seal can be discharged to the drain pipe;
a health monitoring apparatus for monitoring a sample to be monitored;
a water level sensor for monitoring a water level of the water seal; and
a control unit electrically connected to the water level sensor and the health monitoring apparatus, wherein the control unit can control the health monitoring apparatus to turn on based on a monitoring signal of the water level sensor.

47. The health monitoring toilet according to claim 46, wherein the monitoring signal comprises a first water level signal, the first water level signal is a signal monitored by the water level sensor that the water level of the water seal is lower than a first height, and the control unit controls the health monitoring apparatus to turn on based on the first water level signal.

48. The health monitoring toilet according to claim 47, further comprising a spray unit, wherein the spray unit comprises a nozzle and a control valve, the nozzle has a spray channel for supplying water to the water seal, and the control valve is used for controlling the on/off of the spray channel; the control unit is further electrically connected to the control valve, and the control unit controls the control valve to open upon receiving the first water level signal and after the health monitoring apparatus is turned off.

49. The health monitoring toilet according to claim 48, wherein the control unit controls the control valve to open and then close after a delay.

50. The health monitoring toilet according to claim 48, wherein the bottom of the toilet bowl is provided with a first mounting hole, and the health monitoring apparatus is mounted in the first mounting hole.

51. The health monitoring toilet according to claim 50, wherein the nozzle is mounted in the first mounting hole, or the bottom of the toilet bowl is further provided with a second mounting hole, the nozzle is mounted in the second mounting hole, and the spray channel sprays water onto the water seal to form a siphon in the drain pipe to suck water from the water seal.

52. The health monitoring toilet according to claim 48, wherein the water level sensor is mounted on the health monitoring apparatus, or the water level sensor is mounted on the nozzle.

53. The health monitoring toilet according to claim 48, wherein the health monitoring apparatus is a urine test unit or a stool test unit; the control valve is an electromagnetic valve.

54. The health monitoring toilet according to claim 46, wherein the water level sensor is a capacitive sensor, an electrode needle, or a float switch.
